# EUROPEAN PATENT SPECIFICATION

(11) **EP 4 269 581 B1**
(45) Date of publication and mention of the grant of the patent: **11.06.2025**
(21) Application number: 21915214.7
(22) Date of filing: 24.12.2021
(51) Int. Cl.: C12N 15/09, C12Q 1/34, C12Q 1/68, C12Q 1/6876, C12Q 1/6825, C12Q 1/6837

(54) **METHOD AND KIT FOR DETECTING TARGET NUCLEIC ACID FRAGMENT**
VERFAHREN UND KIT ZUM NACHWEIS EINES ZIELNUKLEINSÄUREFRAGMENTS
PROCÉDÉ ET KIT DE DÉTECTION DE FRAGMENT D'ACIDE NUCLÉIQUE CIBLE

(30) Priority: 28.12.2020 JP 2020219481
(43) Date of publication of application: 01.11.2023
(73) Proprietor: RIKEN, Wako-shi, Saitama 351-0198 (JP)
(72) Inventor: WATANABE Rikiya, Wako-shi, Saitama 351-0198 (JP); SHINODA Hajime, Wako-shi, Saitama 351-0198 (JP); MAKINO Asami, Wako-shi, Saitama 351-0198 (JP); IIDA Tatsuya, Wako-shi, Saitama 351-0198 (JP)
(74) Representative: Cabinet Becker et Associés
(86) International application number: PCT/JP2021/048095
(87) International publication number: WO 2022/145354

(56) References cited:
- EP-A1- 3 995 833
- WO-A2-2020/102608
- BRUCH RICHARD ET AL: "CRISPR/Cas13a-Powered Electrochemical Microfluidic Biosensor for Nucleic Acid Amplification-Free miRNA Diagnostics", ADVANCED MATERIALS, vol. 31, no. 51, 30 October 2019 (2019-10-30), XP055899487, DOI: 10.1002/adma.201905311
- RIKIYA WATANABE, NAOKI SOGA, DAISHI FUJITA, KAZUHITO V. TABATA, LISA YAMAUCHI, SOO HYEON KIM, DAISUKE ASANUMA, MAKO KAMIYA, YASUTE: "Arrayed lipid bilayer chambers allow single-molecule analysis of membrane transporter activity", NATURE COMMUNICATIONS, vol. 5, 4519, 1 January 2014 (2014-01-01), pages 1 - 8, XP055217385, DOI: 10.1038/ncomms5519
- GOOTENBERG JONATHAN S., ABUDAYYEH OMAR O., LEE JEONG WOOK, ESSLETZBICHLER PATRICK, DY AARON J., JOUNG JULIA, VERDINE VANESSA, DONG: "Nucleic acid detection with CRISPR-Cas13a/C2c2", SCIENCE, vol. 356, no. 6336, 28 April 2017 (2017-04-28), US , pages 438 - 442, XP055816752, ISSN: 0036-8075, DOI: 10.1126/science.aam9321
- SHINODA HAJIME, TAGUCHI YUYA, NAKAGAWA RYOYA, MAKINO ASAMI, OKAZAKI SAE, NAKANO MASAHIRO, MURAMOTO YUKIKO, TAKAHASHI CHIHARU, TAKA: "Amplification-free RNA detection with CRISPR–Cas13", COMMUNICATIONS BIOLOGY, vol. 4, no. 1, 1 December 2021 (2021-12-01), pages 1 - 7, XP055948638, DOI: 10.1038/s42003-021-02001-8
- RIKIYA WATANABE: "Rapid digital detection technology for novel coronavirus", BIOSCIENCE AND INDUSTRY, vol. 79, no. 5, 10 September 2021 (2021-09-10), JP , pages 427 - 429, XP009538023, ISSN: 0914-8981

## Description

### [Technical Field]

The present invention relates to a method and a kit for detecting a target nucleic acid fragment. Priority is claimed on Japanese Patent Application No. 2020-219481 filed December 28, 2020 in Japan.

### [Background Art]

A technology of detecting a target nucleic acid fragment in a sample with high sensitivity is required. For example, it is known that blood contains free DNA (cell-free DNA, cfDNA) released from cells due to cell death. Blood tumor DNA (circulating tumor DNA, ctDNA), which is DNA derived from cancer cells, is also contained in the cfDNA of cancer patients.

In addition, there is also a demand for testing whether or not a virus causing an infectious disease is present in a sample such as a subject's saliva, throat swab, and nasal swab.

In addition, it is known that various cells secrete membrane vesicles referred to as exosomes, and exosomes are included in biological samples such as saliva, blood, urine, amniotic fluid, and malignant ascites, or in a supernatant of cultured cells. Exosomes contain various proteins, lipids, microRNAs, DNAs, and the like, derived from cells that secrete thereof.

In recent years, researches have been conducted in which cfDNA, microRNA (miRNA) in membrane vesicles such as exosomes, DNA, or the like is applied to early detection of cancer or various other diseases, prediction of an effect of anticancer drugs, diagnosis of predisposition to diseases, diagnosis of genetic diseases, and the like. A technology of detecting a target nucleic acid fragment in a sample with high sensitivity can be applied to such fields as an example.

By the way, an acquired immune mechanism discovered in prokaryotes is referred to as the CRISPR/Cas system, and has been applied to genome editing in recent years. There are a plurality of families of CRISPR/Cas proteins. It is clarified that, among the CRISPR/Cas protein family, a Cas12 and a Cas13 form a three-part complex with a crRNA and a target nucleic acid, and in a case where the target nucleic acid is cleaved, activity of cleaving the surrounding DNA or RNA is exhibited. For example, Non-Patent Documents 1 to 3 report methods for detecting a target nucleic acid fragment with high sensitivity using such activities of the Cas12 and the Cas13.

In addition, Patent Document 1 discloses detection of enzymatic activity at a single-molecule level using a microchamber of a femtoliter order size, Bruch et al. (Advanced Materials, vol. 31, no. 51, 30 October 2019) describe a CRISPR/Cas13a-powered electrochemical microfluidic biosensor for nucleic acid amplification-free miRNA diagnostics, and WO2020/102608 describes methods for generating primers and/or probes for use in analyzing a sample which may comprise a pathogen target sequence, including pan-viral sets of primers and/or probes.

### [Citation List]

### [Patent Document]

[Patent Document 1]
Japanese Unexamined Patent Application, First Publication No. 2004-309405

### [Non-Patent Documents]

[Non-Patent Document 1]
   Gootenberg JS, et al., Nucleic acid detection with CRISPR-Cas13a/C2c2, Science, 356 (6336), 438-442, 2017.
[Non-Patent Document 2]
   Gootenberg JS, et al., Multiplexed and portable nucleic acid detection platform with Cas13, Cas12a, and Csm6, Science, 360 (6387), 439-444, 2018.
[Non-Patent Document 3]
   Chen JS, et al., CRISPR-Cas12a target binding unleashes indiscriminate single-stranded DNase activity, Science, 360 (6387), 436-439, 2018.

### [Summary of Invention]

### [Technical Problem]

By miniaturizing a volume of a reaction space where an enzymatic reaction is performed, as in the microchamber disclosed in Patent Document 1, a detection time of the enzymatic reaction can be shortened. However, in a case where the volume of the reaction space is miniaturized, there is a case where a proportion of the target substance captured in the reaction space decreases, and detection sensitivity is lowered. In addition, the methods disclosed in Non-Patent Documents 1 to 3 require a step of amplifying the target nucleic acid fragment. Accordingly, an object of the present invention is to provide a technology capable of detecting a target nucleic acid fragment with high sensitivity without amplification.

### [Solution to Problem]

The present description includes the following aspects.
[1] A method for detecting a target nucleic acid fragment in a sample, including: step (a) of contacting the sample with a gRNA complementary to the target nucleic acid fragment, a CRISPR/Cas family protein, and a substrate nucleic acid fragment, in which the CRISPR/Cas family protein expresses nuclease activity after forming a three-part complex with the gRNA and the target nucleic acid fragment, the CRISPR/Cas family protein is immobilized on a solid phase, the substrate nucleic acid fragment is labeled with a fluorescent substance and a quencher, in a case where the fluorescent substance cleaved by the nuclease activity of the three-part complex is separated from the quencher, fluorescent light is emitted by irradiation with excitation light, the contact is performed in a reaction space having a volume of 10 aL to 100 pL, thereby forming the three-part complex in a case where the target nucleic acid fragment is present in the sample, cleaving the substrate nucleic acid fragment, and separating the fluorescent substance from the quencher, and step (b) of irradiating the fluorescent substance with the excitation light to detect the fluorescent light, in which the detection of the fluorescent light indicates the presence of the target nucleic acid fragment in the sample, and
   i) in which the step (a) is performed in each well of a well array,
      each well has a first well and a second well arranged at a bottom of the first well and having a smaller capacity than the first well,
      the reaction space is a space inside the second well,
      the CRISPR/Cas family protein is immobilized on an inner surface of the second well,
      the step (a) includes
      step (a1') of introducing the sample and the substrate nucleic acid fragment into each well of the well array in a case where the CRISPR/Cas family protein has formed a two-part complex with the gRNA in advance, and introducing the sample, the gRNA, and the substrate nucleic acid fragment into each well of the well array in a case where the CRISPR/Cas family protein has not formed the two-part complex with the gRNA in advance,
      step (a2') of sealing each well of the well array with a sealing liquid, and step (a3') of replacing the sealing liquid with a water-absorbing organic solvent, thereby dehydrating a content of the wells, reducing a volume, causing accumulation of the content inside the second well, forming the three-part complex in the presence of the target nucleic acid fragment in the content, cleaving the substrate nucleic acid fragment, and separating the fluorescent substance from the quencher, or
   ii) in which the CRISPR/Cas family protein is immobilized on a surface of a particle, the method further comprising, before the step (a): a step of mixing the sample, the gRNA, and the CRISPR/Cas family protein in a container, and forming the three-part complex including the CRISPR/Cas family protein, the gRNA, and the target nucleic acid fragment on the particle.
[2] A method for detecting a target nucleic acid fragment in a sample, including: step (a) of contacting the sample with a gRNA complementary to the target nucleic acid fragment, a CRISPR/Cas family protein, and a substrate nucleic acid fragment, in which the CRISPR/Cas family protein expresses nuclease activity after forming a three-part complex with the gRNA and the target nucleic acid fragment, the CRISPR/Cas family protein is immobilized on a solid phase, the substrate nucleic acid fragment is labeled with a fluorescent substance and a quencher, in a case where the fluorescent substance cleaved by the nuclease activity of the three-part complex is separated from the quencher, fluorescent light is emitted by irradiation with excitation light, the contact is performed in a reaction space having a volume of 10 aL to 100 pL, thereby forming the three-part complex in a case where the target nucleic acid fragment is present in the sample, cleaving the substrate nucleic acid fragment, and separating the fluorescent substance from the quencher, and step (b) of irradiating the fluorescent substance with the excitation light to detect the fluorescent light, in which the detection of the fluorescent light indicates the presence of the target nucleic acid fragment in the sample, and in which the step (a) is performed in each well of a well array, the reaction space is a space inside each well, the CRISPR/Cas family protein is immobilized on an inner surface of the each well, and the step (a) includes step (a1) of introducing the sample and the substrate nucleic acid fragment into each well of the well array in a case where the CRISPR/Cas family protein has formed a two-part complex with the gRNA in advance, and introducing the sample, the gRNA, and the substrate nucleic acid fragment into each well of the well array in a case where the CRISPR/Cas family protein has not formed the two-part complex with the gRNA in advance, and step (a2) of sealing each well of the well array with a sealing liquid, thereby forming the three-part complex in the presence of the target nucleic acid fragment in the well, cleaving the substrate nucleic acid fragment, and separating the fluorescent substance from the quencher.
[3] A method according to [1], in which the sealing liquid is a fluorine-based liquid, a mineral oil, or a linear or branched saturated or unsaturated hydrocarbon having 7 to 17 carbon atoms.
[4] A method according to [1] or [3], in which the water-absorbing organic solvent is a linear or branched saturated or unsaturated aliphatic alcohol having 4 to 11 carbon atoms.
[5] A method according to any one of [1] to [4], in which the CRISPR/Cas family protein is a Cas12 protein or a Cas13 protein.
[6] A method according to any one of [1] to [5], in which 0 or 1 of the target nucleic acid fragment is introduced into the each reaction space.
[7] A method according to any one of [1] to [6], in which the sample is a biological sample and the step (a) is performed without purifying the target nucleic acid fragment from the biological sample.
[8] A kit for detecting a target nucleic acid fragment, including: a substrate having a surface on which a well having a volume of 10 aL to 100 pL is formed; a gRNA complementary to the target nucleic acid fragment; a CRISPR/Cas family protein immobilized on a solid phase; and a substrate nucleic acid fragment, in which the CRISPR/Cas family protein expresses nuclease activity after forming a three-part complex with the gRNA and the target nucleic acid fragment, the substrate nucleic acid fragment is labeled with a fluorescent substance and a quencher, and in a case where the fluorescent substance cleaved by the nuclease activity of the three-part complex is separated from the quencher, fluorescent light is emitted by irradiation with excitation light, and in which the well has a first well and a second well arranged at a bottom of the first well and having a smaller capacity than the first well, and the CRISPR/Cas family protein is immobilized on an inner surface of the second well.
[9] A kit for detecting a target nucleic acid fragment, including: a substrate having a surface on which a well having a volume of 10 aL to 100 pL is formed; a gRNA complementary to the target nucleic acid fragment; a CRISPR/Cas family protein immobilized on a solid phase; and a substrate nucleic acid fragment, in which the CRISPR/Cas family protein expresses nuclease activity after forming a three-part complex with the gRNA and the target nucleic acid fragment, the substrate nucleic acid fragment is labeled with a fluorescent substance and a quencher, and in a case where the fluorescent substance cleaved by the nuclease activity of the three-part complex is separated from the quencher, fluorescent light is emitted by irradiation with excitation light, and in which the CRISPR/Cas family protein is immobilized on a surface of a particle/
[10] A kit for detecting a target nucleic acid fragment in a sample, including: a substrate having a surface on which a well having a volume of 10 aL to 100 pL is formed; a gRNA complementary to the target nucleic acid fragment; a CRISPR/Cas family protein immobilized on a solid phase; and a substrate nucleic acid fragment, in which the CRISPR/Cas family protein expresses nuclease activity after forming a three-part complex with the gRNA and the target nucleic acid fragment, the substrate nucleic acid fragment is labeled with a fluorescent substance and a quencher, and in a case where the fluorescent substance cleaved by the nuclease activity of the three-part complex is separated from the quencher, fluorescent light is emitted by irradiation with excitation light, and in which the CRISPR/Cas family protein is immobilized on an inner surface of the well.
[11] The kit according to [10], in which the CRISPR/Cas family protein is a Cas12 protein or a Cas13 protein.

### [Advantageous Effects of Invention]

According to the present invention, it is possible to provide a technology of capable of performing detection with high sensitivity without amplifying a target nucleic acid fragment.

### [Brief Description of Drawings]

FIG. 1(a) and FIG. 1(b) are schematic diagrams showing a method for detecting a target nucleic acid fragment.
FIG. 2(a) is a top view showing an example of a fluid device. FIG. 2(b) is a cross-sectional view taken along line b-b' in an arrow direction of FIG. 2(a).
FIGS. 3(a) to 3(c) are schematic cross-sectional views showing an example of procedures for carrying out a method for detecting a target nucleic acid fragment.
FIG. 4(a) to 4(d) are schematic diagrams showing a method for immobilizing a CRISPR/Cas family protein on an inner surface of a well.
FIG. 5(a) is a top view showing an example of a well array having a first well and a second well. FIG. 5(b) is a cross-sectional view taken along line b-b' in an arrow direction of FIG. 5(a).
FIG. 6(a) is a top view showing an example of a well array having a first well and a second well. FIG. 6(b) is a cross-sectional view taken along line b-b' in an arrow direction of FIG. 6(a).
FIGS. 7(a) to 7(f) are schematic cross-sectional views showing each step of forming a well array.
FIGS. 8(a) and 8(b) are schematic cross-sectional views showing each step of forming a well array.
FIG. 9 is a photomicrograph of an actually manufactured well array having a first well and a second well.
FIG. 10(a) is a top view showing an example of a fluid device. FIG. 10(b) is a cross-sectional view taken along line b-b' in an arrow direction of FIG. 10(a).
FIGS. 11(a) to 11(c) are schematic diagrams showing a method for detecting a target nucleic acid fragment.
FIGS. 12(a) to 12(d) are schematic diagrams showing a method for detecting a target nucleic acid fragment.
FIGS. 13(a) to 13(d) are representative fluorescence micrographs showing a result of Experimental Example 1.
FIG. 14 is a graph showing a result of Experimental Example 1.
FIG. 15 is a graph showing a result of Experimental Example 2.
FIG. 16 is a fluorescence micrograph showing a result of Experimental Example 3.
FIG. 17 is a graph showing a result of Experimental Example 4.
FIG. 18 is a graph showing a result of Experimental Example 5.
FIG. 19 is a graph showing a result of Experimental Example 6.
FIG. 20 is a graph showing a result of Experimental Example 7.
FIG. 21 is a schematic diagram showing a method for immobilizing a CRISPR/Cas family protein on a surface of a particle.
FIGS. 22(a) and 22(b) are schematic diagrams showing a method for immobilizing a CRISPR/Cas family protein on a surface of a particle.
FIG. 23 is a graph showing a result of Experimental Example 8.
FIG. 24 is a schematic diagram showing a method for detecting a target nucleic acid fragment performed in Experimental Example 9.
FIG. 25 is a fluorescence micrograph showing a result of Experimental Example 9.

### [Description of particular Aspects]

Hereinafter, aspects and embodiments of the present invention will be described in detail with reference to the drawings as the case may be. In the drawings, the same or corresponding parts are denoted by the same or corresponding reference numerals, and overlapping descriptions are omitted. Note that dimensional ratios in each drawing are exaggerated for the sake of explanation, and do not necessarily match the actual dimensional ratios.

### [Method for detecting target nucleic acid fragment]

In one aspect, the present description provides a method for detecting a target nucleic acid fragment in a sample, including step (a) of contacting the sample with a gRNA complementary to the target nucleic acid fragment, a CRISPR/Cas family protein, and a substrate nucleic acid fragment; and a step (b) of irradiating the fluorescent substance with the excitation light to detect the fluorescent light, in which detection of the fluorescent light indicates the presence of the target nucleic acid fragment in the sample.

In the step (a), the CRISPR/Cas family protein expresses nuclease activity after forming a three-part complex with the gRNA and the target nucleic acid fragment. In addition, the CRISPR/Cas family protein is immobilized on a solid phase. In addition, the substrate nucleic acid fragment is labeled with a fluorescent substance and a quencher, and in a case where the fluorescent substance cleaved by the nuclease activity of the three-part complex is separated from the quencher, fluorescent light is emitted by irradiation with excitation light. In addition, the contact of the sample, the gRNA, the CRISPR/Cas family protein, and the substrate nucleic acid fragment is performed in a reaction space having a volume of 10 aL to 100 pL. As a result, the three-part complex is formed in a case where the target nucleic acid fragment is present in the sample, the substrate nucleic acid fragment is cleaved, and the fluorescent substance is separated from the quencher.

Subsequently, in the step (b), fluorescent light is detected in a case where the fluorescent substance is irradiated with excitation light.

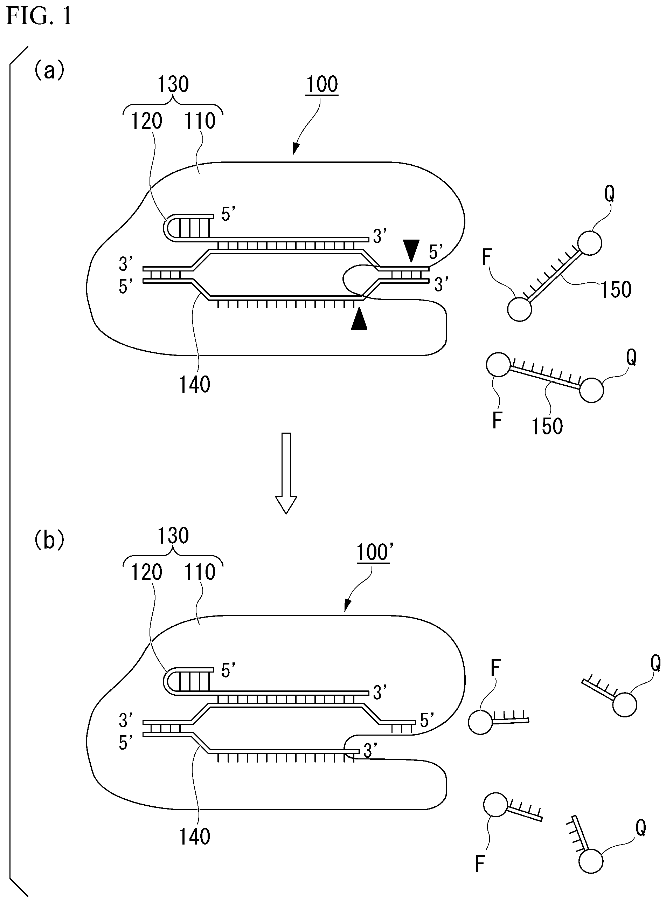

FIGS. 1(a) and 1(b) are schematic diagrams showing the method of the present aspect. FIGS. 1(a) and 1(b) show an example of a case where the CRISPR/Cas family protein is a Cas12a protein.

First, as shown in FIG. 1(a), in step (a), in a case where a Cas12a protein 110 and a gRNA 120 are brought into contact with each other, these bind to form a two-part complex 130. The gRNA 120 partially has a base sequence complementary to a target nucleic acid fragment 140.

Subsequently, in a case where the target nucleic acid fragment 140 in a sample is contacted with the two-part complex 130, the Cas12a protein 110, the gRNA 120, and the target nucleic acid fragment 140 form a three-part complex 100. At this stage, since the Cas12a protein 110 does not express nuclease activity, a substrate nucleic acid fragment 150 is not cleaved. In the examples of FIGS. 1(a) and 1(b), the substrate nucleic acid fragment 150 is a single-stranded DNA fragment labeled with a fluorescent substance F and a quencher Q. Even if the substrate nucleic acid fragment 150 is irradiated with excitation light, fluorescent light is not generated.

In a case where the three-part complex 100 is formed, the Cas12a protein 110 cleaves a target site of the target nucleic acid fragment 140. In FIG. 1(a), the target site of the target nucleic acid fragment 140 is indicated by an arrowhead. FIG. 1(b) is a schematic diagram showing a three-part complex 100' in which the target site of the target nucleic acid fragment 140 is cleaved. As shown in FIG. 1(b), the three-part complex 100' expresses nuclease activity. Then, the substrate nucleic acid fragment 150 existing around the three-part complex 100' is cleaved. As a result, the fluorescent substance F of the substrate nucleic acid fragment 150 is separated from the quencher Q. The fluorescent substance F separated from the quencher Q emits fluorescent light by irradiation with the excitation light.

Subsequently, in step (b), the fluorescent substance F is irradiated with excitation light and fluorescent light is detected. In a case where fluorescent light is detected, it can be determined that the target nucleic acid fragment 140 was present in the sample.

In the method of the present aspect, the CRISPR/Cas family protein is immobilized on a solid phase. As will be described later in examples, by immobilizing the CRISPR/Cas family protein on a solid phase, the detection sensitivity of the target nucleic acid fragment is significantly improved. The CRISPR/Cas family protein, for example, may be immobilized on a surface of a particle, or may be immobilized on an inner surface of the well, which is the reaction space. Details will be described later.

In the method of the present aspect, the sample, the gRNA 120, the CRISPR/Cas family protein 110, and the substrate nucleic acid fragment 150 may be mixed and contacted in any order.

For example, first, the gRNA 120 and the CRISPR/Cas family protein 110 may be brought into contact with each other to form the two-part complex 130 in advance, and then the sample may be brought into contact therewith. In this case, in a case where the target nucleic acid fragment 140 is present in the sample, the target nucleic acid fragment 140 binds to the two-part complex 130 to form a three-part complex 100. After that, the substrate nucleic acid fragment 150 may be brought into contact therewith.

Alternatively, after forming the two-part complex 130, the target nucleic acid fragment 140 and the substrate nucleic acid fragment 150 may be brought into contact simultaneously.

Alternatively, the gRNA 120, the CRISPR/Cas family protein 110, and the sample may be brought into contact simultaneously. Even in this case, in a case where the target nucleic acid fragment 140 is present in the sample, the three-part complex 100 is finally formed. After that, the substrate nucleic acid fragment 150 may be brought into contact therewith.

Alternatively, the sample, the gRNA 120, the CRISPR/Cas family protein 110, and the substrate nucleic acid fragment 150 may be brought into contact therewith simultaneously. Even in this case, in a case where the target nucleic acid fragment 140 is present in the sample, the three-part complex 100 is finally formed, and in the three-part complex 100, in a case where a target site of the target nucleic acid fragment 140 is cleaved, the three-part complex 100 is converted into the three-part complex 100', and expresses nuclease activity, and the substrate nucleic acid fragment 150 is cleaved.

As will be described later in examples, according to the method of the present aspect, a biological sample containing contaminants can be used as a sample, and step (a) can be performed without purifying the target nucleic acid fragment from the biological sample.

More specifically, even if contaminants such as saliva, virus transport buffers, and non-target nucleic acid fragments are present in the sample, the target nucleic acid fragment can be detected almost unaffected by the contaminants.

Currently, in order to detect coronaviruses in saliva, it is necessary to use kits to purify the viral genome RNA in saliva, and this process is very burdensome. On the other hand, according to the method of the present aspect, purification of RNA is not required, and thus the time required for detection of viral genomes can be greatly shortened.

### (Immobilization of CRISPR/Cas family protein-first aspect)

As described above, in the detection method of the present aspect, the CRISPR/Cas family protein is immobilized on a solid phase. In a first embodiment, the CRISPR/Cas family protein may be immobilized on a surface of a particle. Then, before step (a), a step of mixing the sample, the gRNA, and the CRISPR/Cas family protein in a container to form a three-part complex containing the CRISPR/Cas family protein, the gRNA, and the target nucleic acid fragment on the particle may be further included.

Here, as for the immobilization on the surface of the particle, the CRISPR/Cas family protein may be immobilized in a state of being in contact with the particle, or the CRISPR/Cas family protein may be immobilized on the particle via a linker

The particle is not particularly limited, and examples thereof include a resin bead, a glass bead, and the like. Examples of the resin include polyethylene, polypropylene, polystyrene, polycarbonate, cyclic polyolefin, acryl, and the like. The particle may be a magnetic bead. If the particle is a magnetic bead, the particle can be collected using a magnetic stand and the like. The size of the particle can be appropriately selected, and for example, a particle having a diameter of about 0.1 to 100 µm can be used.

In addition, the particle may be colored with a fluorescent pigment and the like. In addition, using a plurality of types of particles each colored with a different dye, a gRNA having a specific base sequence binds to the CRISPR/Cas family protein immobilized on the particle colored with a specific dye. With this, it is possible to recognize the base sequence of the target nucleic acid fragment makes it possible to identify a base sequence of the target nucleic acid fragment by the CRISPR/Cas family protein immobilized on the particle, based on the color of the particle.

Examples of a method of binding particles to the CRISPR/Cas family protein include physical adsorption, a method of performing covalent bond of a functional group present on the surface of the particle and a functional group present on the surface of the CRISPR/Cas family protein using a chemical linker, a method of using hybridization of single-stranded nucleic acid fragments, a method of using avidin-biotin bond, and the like. A combination of these methods may also be used.

Examples of the functional groups in a case of using a chemical linker include hydroxyl group, amino group, thiol group, and the like. Alternatively, for example, the CRISPR/Cas family protein may be immobilized on the surface of the particle using a click reaction using an azide group and an alkyne group and the like. In a case of using avidin-biotin bond, it is possible to bind the particle to the CRISPR/Cas family protein by contacting surface-biotinylated particle, avidin, and the CRISPR/Cas family protein biotinylated using a chemical linker.

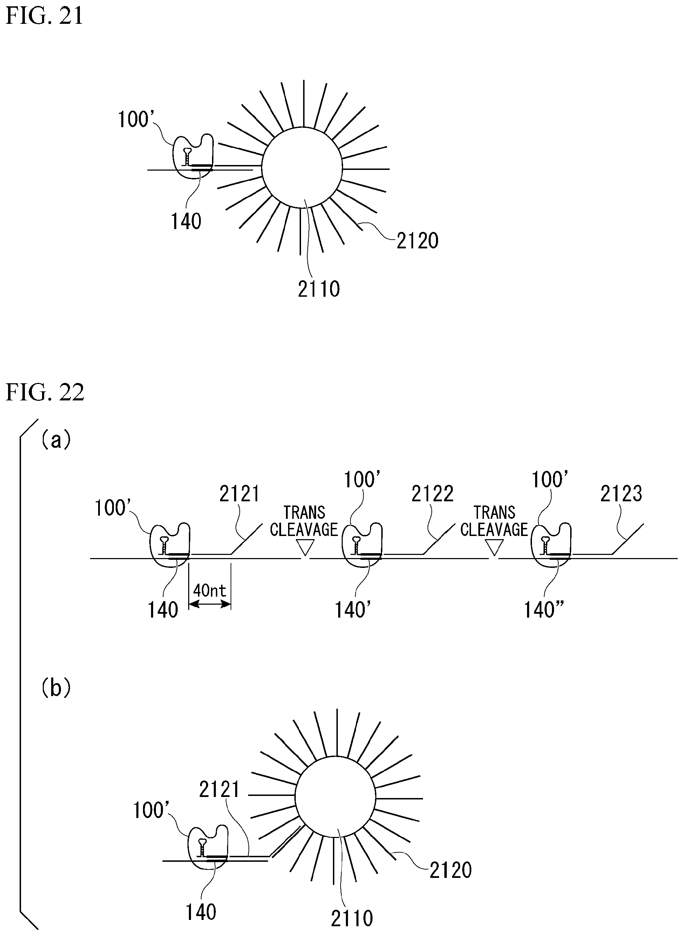

FIGS. 21 and 22 are schematic diagrams showing a method of binding the particle to the CRISPR/Cas family protein using hybridization of single-stranded nucleic acid fragments.

FIG. 21 is a schematic diagram showing one aspect of a method of binding the particle to the CRISPR/Cas family protein. As shown in FIG. 21, a single-stranded DNA fragment 2120 binds to a particle 2110. An end of the single-stranded DNA fragment 2120 is modified with biotin. A surface of the particle 2110 is coated with avidin. Therefore, in a case where the single-stranded DNA fragment 2120 is contacted with the particle 2110, the single-stranded DNA fragment 2120 binds to the surface of the particle 2110 by avidin-biotin bond.

The single-stranded DNA fragment 2120 has a base sequence complementary to a part of a target nucleic acid fragment 140. Therefore, in a case where the particle 2110 to which the single-stranded DNA fragment 2120 binds, a sample containing the target nucleic acid fragment 140, the gRNA, and the CRISPR/Cas family protein are mixed in a container, the three-part complex 100' containing the CRISPR/Cas family protein, the gRNA, and the target nucleic acid fragment 140 are immobilized on the particle 2110 by hybridizing a part of the target nucleic acid fragment 140 with the single-stranded DNA fragment 2120.

FIGS. 22(a) and 22(b) are schematic diagrams showing another aspect for binding the particle to the CRISPR/Cas family protein.

Compared with the particle shown in FIG. 21, the particle shown in FIG. 22(b) is different mainly in a point that the three-part complex 100' is immobilized on the particle 2110 by hybridizing a part of the target nucleic acid fragment 140, the single-stranded DNA fragment 2121, and the single-stranded DNA fragment 2120.

FIG. 22(a) shows a state in which the number of molecules of the three-part complex formed on one molecule of the target nucleic acid fragment is increased by using a plurality of types of gRNAs for one type of the target nucleic acid fragment. As described above, with this, it is possible to increase the number of molecules of the three-part complex per molecule of the target nucleic acid fragment, and thus it is possible to further enhance detection sensitivity of the target nucleic acid fragment.

In an example of FIG. 22(a), the three-part complex 100' that recognizes each of target nucleic acid fragments (target sequences) 140, 140', and 140" binds to one molecule of a long nucleic acid fragment. In addition, single-stranded nucleic acid fragments 2121, 2122, and 2123 having base sequences complementary thereto are hybridized in the vicinity of each of target nucleic acid fragments (target sequences) 140, 140', and 140". In FIG. 22(a), regions of the single-stranded nucleic acid fragments 2121, 2122, and 2123 that do not hybridize with long nucleic acid fragments have base sequences complementary to single-stranded nucleic acid fragment 2120 binding to the particle 2110.

As shown in FIG. 22(a), regions of the single-stranded nucleic acid fragments 2121, 2122, and 2123 hybridize with the long nucleic acid fragments are protected from the nuclease activity of the three-part complex 100', and thus are not cleaved. On the other hand, in the portion indicated by the arrowhead in FIG. 22(a), the long nucleic acid fragment is cleaved (trans cleavage) by the nuclease activity of the three-part complex 100'. As a result, each of a complex of the three-part complex 100' and the single-stranded nucleic acid fragment 2121, a complex of the three-part complex 100' and the single-stranded nucleic acid fragment 2122, and a complex of the three-part complex 100' and the single-stranded nucleic acid fragment 2123 is excised from the long nucleic acid fragment.

Subsequently, as shown in FIG. 22(b), the complex of the excised three-part complex 100' and the single-stranded nucleic acid fragment 2121 is hybridized with the single-stranded DNA fragment 2120 binding to the particle 2110, and thus is immobilized on the particle 2110. Although not shown, the complex of the three-part complex 100' and the single-stranded nucleic acid fragment 2122 and the complex of the three-part complex 100' and the single-stranded nucleic acid fragment 2123 are also similarly hybridized with the single-stranded DNA fragment 2120, and thus is immobilized on the particle 2110.

As described above, the CRISPR/Cas family protein can be immobilized on the particles 2110. The order of mixing the particles 2110, the single-stranded nucleic acid fragment 2120, a sample containing the target nucleic acid fragment 140, the gRNA, the CRISPR/Cas family protein, and the single-stranded nucleic acid fragments 2121, 2122, and 2123 is not limited.

For example, in the example of FIG. 21, the single-stranded nucleic acid fragment 2120 may be mixed in the sample containing the target nucleic acid fragment 140, this may be hybridized, and then the gRNA and the CRISPR/Cas family protein may be mixed therein, and finally particles 2110 may be mixed therein. In addition, the gRNA and the CRISPR/Cas family protein may form a two-part complex in advance.

Alternatively, the sample containing the target nucleic acid fragment 140, the gRNA, and the CRISPR/Cas family protein may be mixed to form a three-part complex, and then the particles 2110 to which the single-stranded DNA fragment 2120 binds may be mixed therein.

In addition, in examples of FIGS. 21(a) and 21(b), the single-stranded nucleic acid fragments 2121, 2122, and 2123 may be mixed in a sample containing a long target nucleic acid, this may be hybridized, and then the gRNA and the CRISPR/Cas family protein may be mixed therein, and subsequently the particles 2110 to which the single-stranded DNA fragments 2120 bind may be mixed therein.

Alternatively, the single-stranded nucleic acid fragments 2120, 2121, 2122, 2123 may be mixed in a sample containing a long target nucleic acid, this may be hybridized, the gRNA and the CRISPR/Cas family protein may be mixed, and finally particles 2110 may be mixed therein. In addition, the gRNA and the CRISPR/Cas family protein may form a two-part complex in advance.

In addition, if the particle 2110 is a magnetic bead, the particle can be collected using a magnetic stand and the like. In addition, it is also easy to wash the particle.

A container for mixing the sample, the gRNA, the CRISPR/Cas family protein, and the like is not particularly limited, and for example, a plastic tube, a well array, and the like can be used.

According to the present method, by immobilizing the CRISPR/Cas family protein on a solid phase, it is possible to efficiently bind the target nucleic acid fragment present at a low concentration in the sample to the CRISPR/Cas family protein and to form a three-part complex. Subsequently, the particle may be distributed into a minute reaction space and contacted with the substrate nucleic acid fragment.

According to the present method, the detection sensitivity of the target nucleic acid fragment is significantly improved. That is, by immobilizing the CRISPR/Cas family protein on the solid phase, it is possible to concentrate the target nucleic acid fragment in the sample.

### (Sample)

The sample is not particularly limited and can be appropriately selected depending on the purpose, and examples thereof include biological samples such as saliva, blood, urine, amniotic fluid, and malignant ascites, throat swab, and nasal swab, or a supernatant of cultured cells.

### (Target nucleic acid fragment)

Examples of the target nucleic acid fragment in a sample include viral genomes, cfDNA, ctDNA, microRNA, exosome-derived DNA, and the like. For example, by using a nucleic acid fragment containing an oncogene hotspot region as a target nucleic acid fragment, it is possible to detect oncogene mutations contained in a sample.

In a case where the CRISPR/Cas family protein is Cas12 protein, it is possible to detect a double-stranded DNA fragment as the target nucleic acid fragment. In addition, in a case where the CRISPR/Cas family protein is Cas13 protein, it is possible to detect a single-stranded RNA fragment or a single-stranded DNA fragment as the target nucleic acid fragment.

### (gRNA)

In the method of the present embodiment, a guide RNA (gRNA) is not particularly limited as long as the guide RNA can be used for the CRISPR/Cas family protein, the guide RNA may be a complex of CRISPR RNA (crRNA) and transactivating CRISPR RNA (tracrRNA), may be a single gRNA (sgRNA) obtained by combining tracrRNA and crRNA, or may be crRNA alone.

In a case where the used CRISPR/Cas family protein is the Cas12a protein, the crRNA can be, for example, the following base sequence. First, a base sequence obtained by removing a protospacer adjacent motif (PAM) sequence from a target base sequence is defined as a spacer base sequence. Subsequently, a base sequence obtained by linking a scaffold sequence to a 3' end of the spacer base sequence is provided, and a complementary strand thereof is used as the base sequence of crRNA.

For example, in a case where the base sequence obtained by removing the PAM sequence from the target base sequence is "5'-GCCAAGCGCACCTAATTTCC-3'" (SEQ ID NO: 1), the base sequence of crRNA for Cas12a protein can be "5'-AAUUUCUACUAAGUGUAGAUGGAAAUUAGGUGCGCUUGGC-3'" (SEQ ID NO: 2).

In a case where the used CRISPR/Cas family protein is a Cas13a protein, the crRNA can be, for example, the following base sequence. First, a base sequence obtained by linking a scaffold sequence to a 3' end of the base sequence complementary to the target base sequence is provided, and the complementary strand thereof is used as the base sequence of crRNA.

For example, in a case where the target base sequence is "5'-AUGGAUUACUUGGUAGAACAGCAAUCUA-3'" (SEQ ID NO: 3), the base sequence of crRNA for Cas13a protein can be "5'-GAUUUAGACUACCCCAAAAACGAAGGGGACUAAAACUAGAUUGCUGUUCU ACCAAGUAAUCCAU-3'" (SEQ ID NO: 4).

A target base sequence is a partial base sequence of the base sequence of the target nucleic acid fragment. At least one type of the target base sequence is set in one type of the target nucleic acid fragment. By setting a plurality of types of target base sequences in one type of target nucleic acid fragment, it is possible to form a three-part complex of a plurality of molecules on one molecule of the target nucleic acid fragment.

That is, by using a plurality of types of gRNAs for one type of the target nucleic acid fragment, it is possible to increase the number of molecules of the three-part complex formed on one molecule of the target nucleic acid fragment. As a result, it becomes possible to increase the number of molecules of the three-part complex expressing nuclease activity per molecule of the target nucleic acid fragment, and to further increase the detection sensitivity of the target nucleic acid fragment.

Therefore, in one aspect, the present description provides the above-described method of detecting a target nucleic acid fragment to increase the detection sensitivity, including a step of forming a plurality of molecules of a three-part complex on one molecule of a target nucleic acid fragment by using a plurality of types of gRNAs.

### (CRISPR/Cas family protein)

In the method of the present aspect, any CRISPR/Cas family protein can be used as long as the CRISPR/Cas family protein can express nuclease activity after forming a three-part complex with a gRNA and a target nucleic acid fragment. As described above, more precisely, a three-part complex is formed, a CRISPR/Cas family protein cleaves a target nucleic acid fragment, and then nuclease activity is expressed.

Examples of such a CRISPR/Cas family protein include the Cas12 protein, the Cas13 protein, and the like. In the present specification, the Cas12 protein and Cas13 protein may be Cas12 protein, Cas13 protein, orthologs of these proteins, variants of these proteins, and the like.

More specific examples of the CRISPR/Cas family protein that can be used in the method of the present aspect include Lachnospiraceae bacterium ND2006-derived Cas12a protein (LbCas12a, UniProtKB Accession Number: A0A182DWE3), Acidaminococcus sp.-derived Cas12a protein (AsCas12a, UniProtKB Accession Number: U2UMQ6), Francisella tularensis subsp. novicida-derived Cas12a protein (FnCas12a, UniProtKB Accession Number: A0Q7Q2), Alicyclobacillus acidoterrestris-derived Cas12b protein (AaCas12b, UniProtKB Accession Number: T0D7A2), Leptotrichia wadei-derived Cas13a protein (LwaCas13a, NCBI Accession Number: WP_021746774.1), Lachnospiraceae bacterium NK4A179-derived Cas13a protein (LbaCas13a, NCBI Accession Number: WP_022785443.1), Leptotrichia buccalis C-1013-b-derived Cas13a protein (LbuCas13a, NCBI Accession Number: WP_015770004.1), Bergeyella zoohelcum-derived Cas13b protein (BzoCas13b, NCBI Accession Number: WP_002664492), Prevotella intermedia-derived Cas13b protein (PinCas13b, NCBI Accession Number: WP_036860899), Prevotella buccae-derived Cas13b protein (PbuCas13b, NCBI Accession Number: WP_004343973), Alistipes sp. ZOR0009-derived Cas13b protein (AspCas13b, NCBI Accession Number: WP_047447901), Prevotella sp. MA2016-derived Cas13b protein (PsmCas13b, NCBI Accession Number: WP_036929175), Riemerella anatipestifer-derived Cas13b protein (RanCas13b, NCBI Accession Number: WP_004919755), Prevotella aurantiaca-derived Cas 13b protein (PauCas13b, NCBI Accession Number: WP_025000926), Prevotella saccharolytica-derived Cas13b protein (PsaCas13b, NCBI Accession Number: WP_051522484), Prevotella intermedia-derived Cas13b protein (Pin2Cas13b, NCBI Accession Number: WP_061868553), Capnocytophaga canimorsus-derived Cas13b protein (CcaCas13b, NCBI Accession Number: WP_013997271), Porphyromonas gulae-derived Cas13b protein (PguCas13b, NCBI Accession Number: WP_039434803), Prevotella sp. P5-125-derived Cas13b protein (PspCas13b, NCBI Accession Number: WP_044065294), Porphyromonas gingivalis-derived Cas13b protein (PigCas13b, NCBI Accession Number: WP_053444417), Prevotella intermedia-derived Cas13b protein (Pin3Cas13b, NCBI Accession Number: WP_050955369), Enterococcus italicus-derived Csm6 protein (EiCsm6, NCBI Accession Number: WP_007208953.1), Lactobacillus salivarius-derived Csm6 protein (LsCsm6, NCBI Accession Number: WP_081509150.1), Thermus thermophilus-derived Csm6 protein (TtCsm6, NCBI Accession Number: WP_011229148.1), and the like.

In the method of the present aspect, the CRISPR/Cas family protein may be a mutant of the above-described Cas family protein. As a mutant, for example, a mutant with increased nuclease activity after forming a three-part complex and the like can be used.

### (Substrate nucleic acid fragment)

The substrate nucleic acid fragment is labeled with a fluorescent substance and a quencher, and in a case where the fluorescent substance cleaved by the nuclease activity of the three-part complex is separated from the quencher, fluorescent light is emitted by irradiation with excitation light.

The substrate nucleic acid fragment may be appropriately selected according to substrate specificity of the used CRISPR/Cas family protein. For example, the Cas12 protein cleaves a single-stranded DNA as a substrate. Therefore, in a case of using the Cas12 protein, a single-stranded DNA may be used as the substrate nucleic acid fragment. In addition, the Cas13 protein cleaves a single-stranded RNA as a substrate. Therefore, in a case of using the Cas13 protein, a single-stranded RNA may be used as the substrate nucleic acid fragment.

As for combination of the fluorescent substance and the quencher, a combination that can quench the fluorescent light of the fluorescent substance in a case of being brought close to each other is used. For example, in a case of using FAM, HEX, and the like as the fluorescent substance, Iowa Black FQ (IDT), TAMRA, or the like can be used as the quencher.

By the way, depending on the type of the Cas13 protein, it is known that there is specificity in the base sequence of the single-stranded RNA to be cleaved. Specifically, for example, each of a LwaCas13a protein, a CcaCas13b protein, a LbaCas13a protein, and a PsmCas13b protein is reported to recognize and cleave AU, UC, AC, and GA base sequences in substrate nucleic acid fragments.

Therefore, in the method of the present aspect, for example, by using the LwaCas13a protein, the CcaCas13b protein, the LbaCas13a protein, and the PsmCas13b protein, as the CRISPR/Cas family protein, each different gRNA is combined with each CRISPR/Cas family protein, as a gRNA, and by using a single-stranded RNA containing AU, UC, AC, and GA base sequences, as a substrate nucleic acid fragment, and further labeling each substrate nucleic acid fragment with different fluorescent pigments identifiable with each other, it is possible to detect four types of target nucleic acid fragments in one reaction space. That is, it is possible to perform multicolor detection.

### (Reaction space)

As a technique for detecting a target substance such as a target nucleic acid fragment with high accuracy, a technology of performing an enzymatic reaction in a large number of minute reaction spaces has been examined. These techniques are referred to as digital measurement. In digital measurement, a sample is divided into a very large number of minute reaction spaces to detect a signal.

Then, the signal from each reaction space is binarized, only the presence or absence of the target substance is discriminated, and the number of molecules of the target substance is measured. According to the digital measurement, compared to ELISA, a real-time PCR, and the like in the related art, it is possible to significantly improve detection sensitivity and quantification.

The method of the present aspect is preferably performed by digital measurement. More specifically, contact between the sample, the CRISPR/Cas family protein, the gRNA, and the substrate nucleic acid fragment is performed by dividing the contact of the substrate nucleic acid fragment into minute reaction spaces. The volume per reaction space is 10 aL to 100 pL, for example, may be 10 aL to 10 pL, for example, may be 10 aL to 1 pL, for example, may be 10 aL to 100 fL, and for example, may be 10 aL to 10 fL. By setting the reaction space in the above range, it is possible to detect the presence thereof with high sensitivity without amplifying the target nucleic acid fragment.

By performing the method of the present aspect under conditions of introducing 0 or 1 of the target nucleic acid fragment per reaction space, it is possible to perform digital measurement. That is, it is possible to cause the number of the reaction space in which a signal is detected to correspond to the number of molecules of the target nucleic acid fragment in the sample.

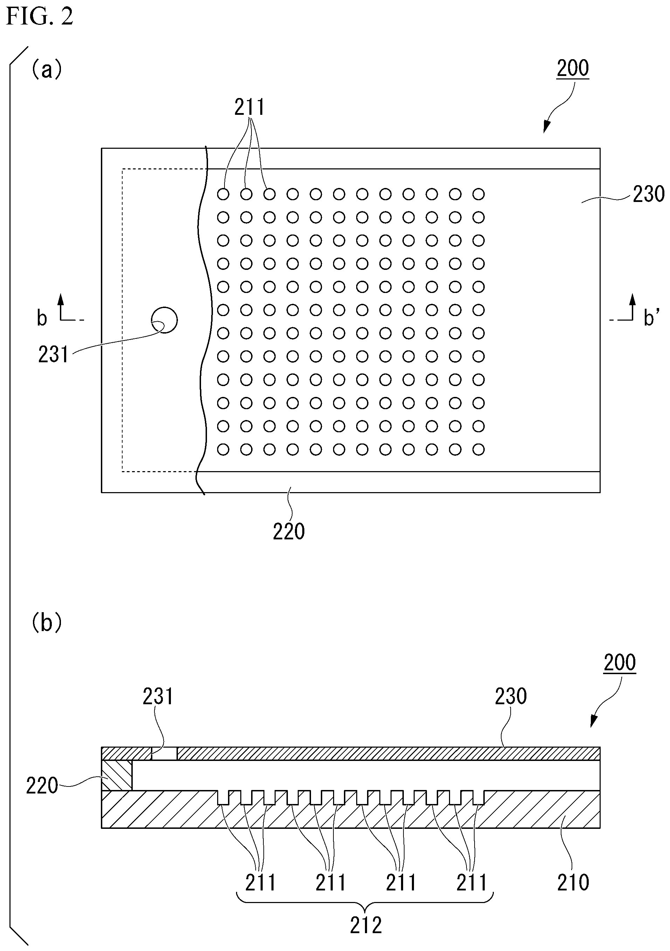

The reaction space may be a droplet, for example. Alternatively, the reaction space may be a well formed on a substrate. FIG. 2(a) is a top view showing an example of a fluid device having a substrate on which wells each having a volume of 10aL to 100pL per well are formed. FIG. 2(b) is a cross-sectional view taken along line b-b' in an arrow direction of FIG. 2(a).

As shown in FIGS. 2(a) and 2(b), a fluid device 200 includes a substrate 210 on which a well 211 having a volume of 10 aL to 100 pL is formed, a spacer 220, and a lid member 230 having a liquid inlet 231 formed thereon. A plurality of the wells 211 are present to form a well array 212. A space between the substrate 210 and the lid member 230 functions as a flow path through which a sample, a gRNA, a CRISPR/Cas family protein, a substrate nucleic acid fragment, and the like flow.

A shape of the well is not particularly limited as long as the volume is within the range described above, and for example, may be cylindrical, polyhedral formed by a plurality of faces (for example, rectangular parallelepiped, hexagonal, octagonal, and the like), and the like.

In the fluid device 200, the plurality of the wells 211 of the same shape and size form a well array 212. Here, "same shape and same size" may be the same shape and the same capacity to the extent required for performing digital measurement, and variations to the extent of manufacturing error are allowed.

### (Detection method)

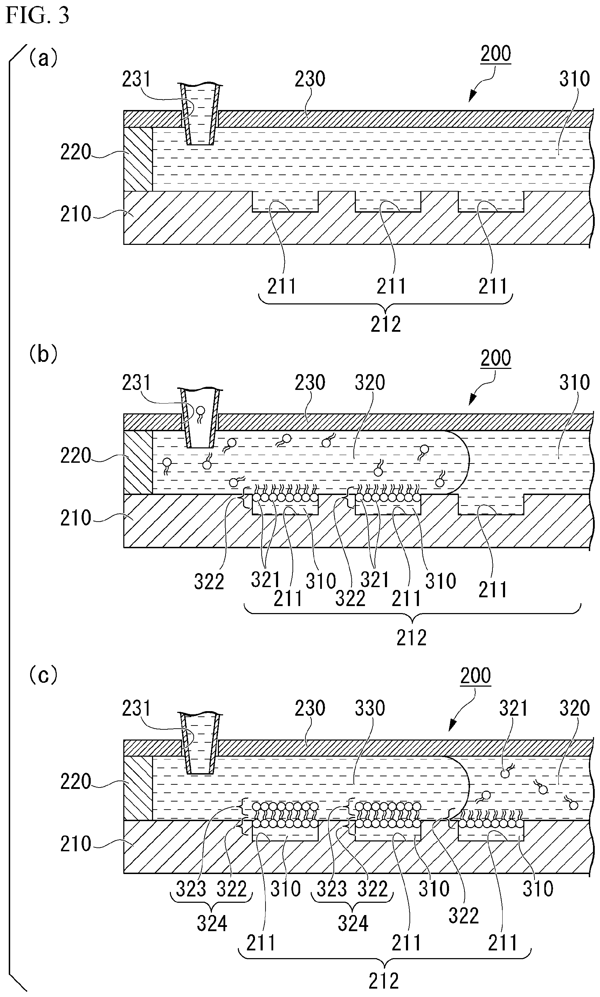

FIGS. 3(a) to 3(c) are schematic cross-sectional views showing an example of the procedure for carrying out the method of the present aspect using the fluid device 200. First, a sample, a gRNA, and a CRISPR/Cas family protein are mixed to form a three-part complex. Subsequently, the three-part complex and the substrate nucleic acid fragment are mixed to prepare an assay solution 310, and the assay solution 310 is immediately introduced through the liquid inlet 231 of the fluid device 200. As a result, as shown in FIG. 3(a), the inside of the well 211 and the space between the substrate 210 and the lid member 230 are filled with the assay solution 310.

Subsequently, as shown in FIG. 3(b), a sealant 320 is introduced through the liquid inlet 231. In an example of FIG. 3(b), an organic solvent containing a lipid 321 is used as the sealant 320. As the lipid 321, natural lipids derived from soybeans, E. coli, and the like and artificial lipids such as dioleoylphosphatidylethanolamine (DOPE) and dioleoylphosphatidylglycerol (DOPG) can be used. As an organic solvent in this case, hexadecane or chloroform can be used. In a case where the sealant 320 is introduced, an opening portion of the well 211 is sealed with a first lipid membrane 322 in a state in which the inside of the well 211 is filled with the assay solution 310. A hydrophilic group of each lipid 321 forming the first lipid membrane 322 faces the well 211 side. As a result, each well 211 becomes an independent reaction space. In this state, the well array 212 is irradiated with excitation light to measure the fluorescent light.

In addition, a lipid membrane can be further laminated on the first lipid membrane 322 to form a lipid bilayer membrane. In this case, as shown in FIG. 3(c), a membrane-forming aqueous solution 330 for forming a lipid bilayer membrane 324 is introduced through the liquid inlet 231. As the composition of the membrane-forming aqueous solution 330, for example, a 10 mM pH buffer solution (pH 5 to 9), a 10 mM sodium chloride aqueous solution, and the like can be used. In a case where the membrane-forming aqueous solution 330 is introduced, a second lipid membrane 323 is laminated on the first lipid membrane 322 to form a lipid bilayer membrane 324. As a result, each well 211 becomes an independent reaction space. In this state, the well array 212 is irradiated with excitation light to measure the fluorescent light.

In a case where the opening portion of the well 211 is sealed with the lipid bilayer membrane 324, lipid vesicles can be fused to the lipid bilayer membrane 324. For example, by fusing exosomes to the lipid bilayer membrane 324, the content of the exosomes can be released inside the well 211.

Therefore, for example, in the well 211, the gRNA, the CRISPR/Cas family protein, and the substrate nucleic acid fragment may be first sealed to seal the opening portion of the well 211 with the lipid bilayer membrane 324, and then exosomes may be contacted with the lipid bilayer membrane 324 as a sample.

As a result, the exosomes are fused to the lipid bilayer membrane 324 and the content of the exosomes is released inside the well 211. In a case where the target nucleic acid fragment is present in the content of the exosomes, a three-part complex is formed inside the well 211, the substrate nucleic acid fragment is cleaved, and fluorescent light is detected by irradiation with excitation light.

### (Immobilization of CRISPR/Cas family proteins-second aspect)

As described above, in the detection method of the present aspect, the CRISPR/Cas family protein is immobilized on a solid phase. In a second aspect, the CRISPR/Cas family protein may be immobilized on the inner surface of the well.

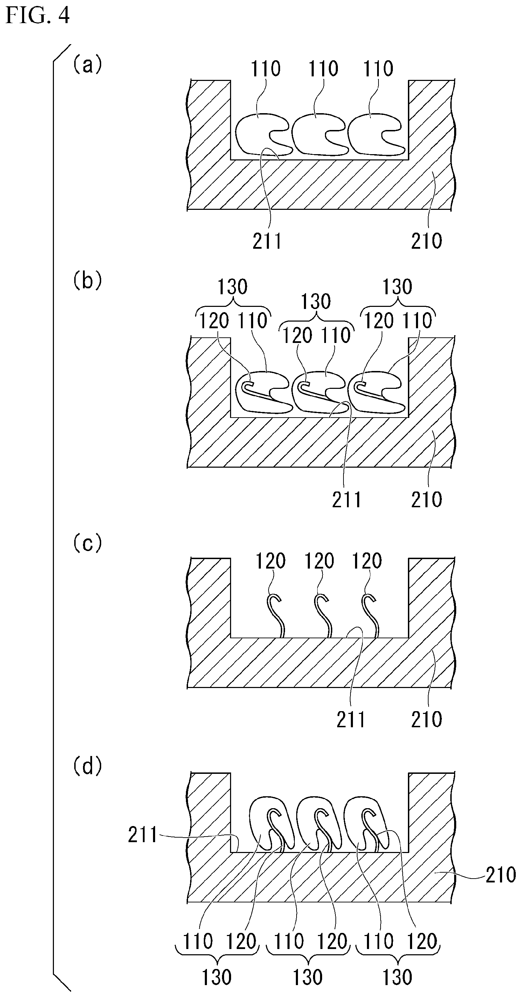

As shown in FIG. 4(a), in the method of the present aspect, the CRISPR/Cas family protein 110 may be immobilized on the inner surface of well 211 in advance. Here, as shown in FIG. 4(b), the CRISPR/Cas family protein 110 may bind to the gRNA 120 to form a two-part complex 130. As a result, it is possible to improve the possibility that the three-part complex 100 is present inside the well 211 in a case where the sample is brought into contact with the well 211, and to dramatically improve the detection sensitivity.

Examples of a method of immobilizing the CRISPR/Cas family protein on the inner surface of the well 211 include physical adsorption, a method of performing covalent bond of a functional group present on the inner surface of the well 211 and a functional group present on the surface of the CRISPR/Cas family protein using a chemical linker, a method of using avidin-biotin bond, and the like. Examples of the functional groups in a case of using a chemical linker include hydroxyl group, amino group, thiol group, and the like. Alternatively, for example, the CRISPR/Cas family protein may be immobilized on the inner surface of the well 211 using a click reaction and the like using an azide group and an alkyne group. In a case of using avidin-biotin bond, the inner surface of the well 211 is biotinylated in advance, and avidin is contacted with the biotinylated CRISPR/Cas family protein using a chemical linker so that the CRISPR/Cas family protein can bind to the inner surface of the well 211.

Alternatively, as shown in FIG. 4(c), gRNA 120 may be immobilized on the inner surface of well 211. Here, gRNA 120 may be added with an additional sequence that functions as a linker.

As a result, as shown in FIG. 4(d), in a case where the CRISPR/Cas family protein 110 is introduced into the well 211, the CRISPR/Cas family protein 110 binds to the gRNA 120 to form the two-part complex 130, and is immobilized on the inner surface of the well 211.

In some cases, there is a case where a well on which a gRNA is immobilized is easier to store than a well on which a protein is immobilized.

In the method of the present aspect, step (a) may be performed in each well of the well array. The reaction space in which the sample, the gRNA, the CRISPR/Cas family protein, and the substrate nucleic acid fragment are brought into contact may be a space inside each well. In addition, the CRISPR/Cas family protein may be immobilized on the inner surface of each well. Step (a) may have the following steps (a1) and (a2).

First, in step (a1), in a case where the CRISPR/Cas family protein has formed a two-part complex with the gRNA in advance, a sample and a substrate nucleic acid fragment are introduced into each well of the well array. In addition, in a case where the CRISPR/Cas family protein has not formed a two-part complex with the gRNA in advance, a sample, a gRNA, and a substrate nucleic acid fragment are introduced into each well of the well array.

Subsequently, in step (a2), each well of the well array is sealed with a sealing liquid. The sealing liquid will be described later. As a result, the three-part complex is formed in a case where the target nucleic acid fragment is present in the well, the substrate nucleic acid fragment is cleaved, and the fluorescent substance is separated from the quencher.

Then, in step (b), the fluorescent substance is irradiated with excitation light. As a result, in a case where the fluorescent light is detected, it can be determined that the target nucleic acid fragment is present in the sample.

Alternatively, in the method of the present aspect, step (a) may be performed in each well of the well array. Each well may have a first well and a second well arranged at a bottom of the first well and having a smaller capacity than the first well. The reaction space in which the sample, the gRNA, the CRISPR/Cas family protein, and the substrate nucleic acid fragment are brought into contact may be a space inside the second well. In addition, the CRISPR/Cas family protein may be immobilized on the inner surface of the second well. Step (a) may have the following steps (a1'), (a2'), and (a3').

First, in step (a1'), in a case where the CRISPR/Cas family protein has formed a two-part complex with the gRNA in advance, a sample and a substrate nucleic acid fragment are introduced into each well of the well array. In addition, in a case where the CRISPR/Cas family protein has not formed a two-part complex with the gRNA in advance, a sample, a gRNA, and a substrate nucleic acid fragment are introduced into each well of the well array.

Subsequently, in step (a2'), each well of the well array is sealed with a sealing liquid. The sealing liquid will be described later.

Subsequently, in step (a3'), the sealing liquid is replaced with a water-absorbing organic solvent. The water-absorbing organic solvent will be described later. As a result, the content of the well is dehydrated, the volume is reduced, the content accumulates inside the second well, the three-part complex is formed in a case where the target nucleic acid fragment is present in the content, the substrate nucleic acid fragment is cleaved, and the fluorescent substance is separated from a quencher.

Then, in step (b), the fluorescent substance is irradiated with excitation light. As a result, in a case where the fluorescent light is detected, it can be determined that the target nucleic acid fragment is present in the sample.

### (Well array)

Here, an array of the wells having a first well and a second well will be described. As described above, each well of the well array has a first well and a second well arranged at a bottom of the first well and having a smaller capacity than the first well, and in a case where the volume of the content of the first well becomes small, the content may accumulate in the second well.

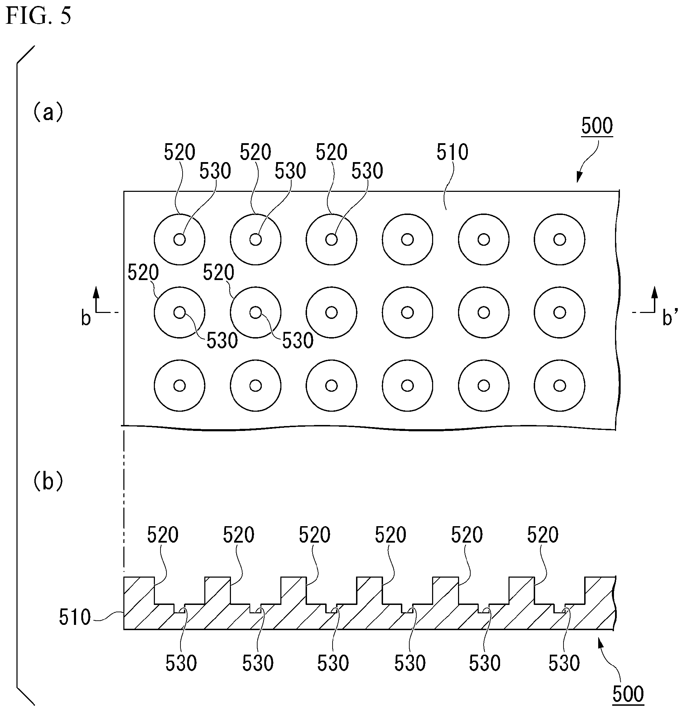

FIGS. 5(a) and 5(b) are schematic diagrams showing an example of a well array having a first well and a second well. FIG. 5(a) is a top view, and FIG. 5(b) is a cross-sectional view taken along line b-b' in an arrow direction of FIG. 5(a).

As shown in FIGS. 5(a) and 5(b), a well array 500 is formed on one surface of a substrate 510. Each well of the well array 500 has a first well 520 and a second well 530 arranged at a bottom of the well 520 and having a smaller capacity than the well 520. As will be described below, in a case where the volume of a content of the first well 520 becomes small, the content accumulates in the second well 530.

That is, by capturing a target substance in the large-capacity well 520 and performing detection in the small-capacity well 530, it is possible to detect the target substance with high sensitivity and a time required for detection can be also shortened.

The ratio of the volume of the first well 520 to the second well 530 (volume of well 520: volume of well 530) may be 10 : 1 to 1,000,000 : 1.

Alternatively, the volume of the first well 520 may be 1 to 1,000 pL and the volume of the second well 530 may be 0.1 to 1,000 fL.

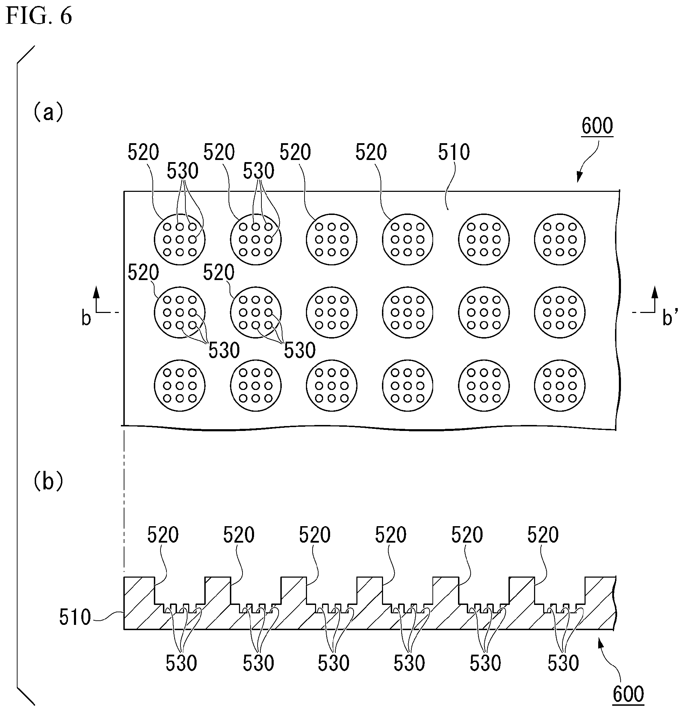

FIGS. 6(a) and 6(b) are schematic diagrams showing another example of a well array having a first well and a second well. FIG. 6(a) is a top view, and FIG. 6(b) is a cross-sectional view taken along line b-b' in an arrow direction of FIG. 6(a).

As shown in FIGS. 6(a) and 6(b), a well array 600 is formed on one surface of a substrate 510. Like the well array 600, a plurality of the second wells 530 may be arranged per the first well 520. Even in this case, in a case where the volume of the content of the first well 520 becomes small, the content accumulates in the second well 530.

A shape of the first well 520 and the second well 530 is not particularly limited, and for example, may be cylindrical, polyhedral formed by a plurality of faces (for example, rectangular parallelepiped, hexagonal, octagonal, and the like), and the like.

The plurality of the first wells 520 preferably have the same shape and same size, and the plurality of the second wells 530 preferably have the same shape and same size. Here, "same shape and same size" may be the same shape and the same capacity to the extent required for performing digital measurement, and variations to the extent of manufacturing error are allowed.

### (Method of manufacturing well array)

An example of a method of manufacturing a well array having a first well and a second well is described using a well array 600 as an example.

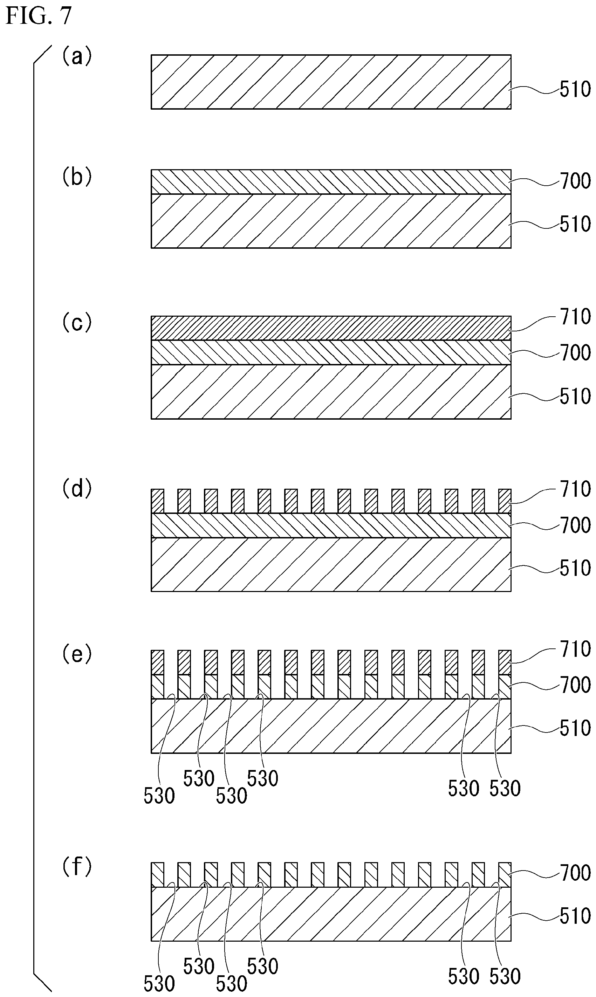

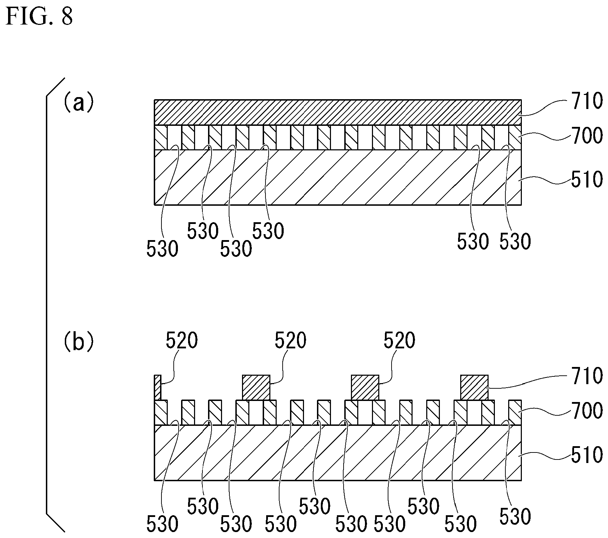

FIGS. 7(a) to 7(f) and FIGS. 8(a) and 8(b) are schematic cross-sectional views showing each step of manufacturing of the well array 600. First, as shown in FIG. 7(a), a film 700 is laminated on a surface of the substrate 510.

Examples of the material for the substrate 510 include glass, resin, and the like. Examples of the resin include polyethylene, polypropylene, polystyrene, polycarbonate, cyclic polyolefin, acryl, and the like.

In particular, polycarbonate is also used as a material for CDs and DVDs, which can be mass-produced at a low cost, and is suitable from a viewpoint of manufacturing a well array at a low cost. The inventors have clarified that in a case of using polycarbonate as a material of the substrate 510, a refractive index of light is close to that of glass, and thus polycarbonate is preferable when detecting fluorescent light with a microscope.

Examples of the material for the film 700 include fluorine resin, cyclic polyolefin, silicone resin, and the like.

Subsequently, as shown in FIG. 7(c), a resist film 710 is laminated on a surface of the film 700. Subsequently, using a well array-pattern mask, the resist film 710 is exposed by irradiation with active energy rays with an exposure machine.
Subsequently, development is performed with a developing solution to remove a portion of the resist film 710 forming the well, as shown in FIG. 4(d).

Subsequently, as shown in FIG. 7(e), the film 700 masked with the resist film 710 is etched to form a second well 530 in the film 700.

Subsequently, as shown in FIG. 7(f), the substrate is washed to remove the resist film 710, and thereby an array of the wells 530 is obtained. Through the steps up to this point, a well array of the second wells is obtained. That is, the array of minute wells manufactured by the steps up to this point can also be used for detection of the target nucleic acid fragment.

Subsequently, the well array of the first wells is stacked on the well array of the second wells by the following steps. Subsequently, as shown in FIG. 8(a), the resist film 710 is laminated again on the array of the wells 530 obtained in FIG. 7(f). As the resist film 710, a sheet-type resist can be preferably used.

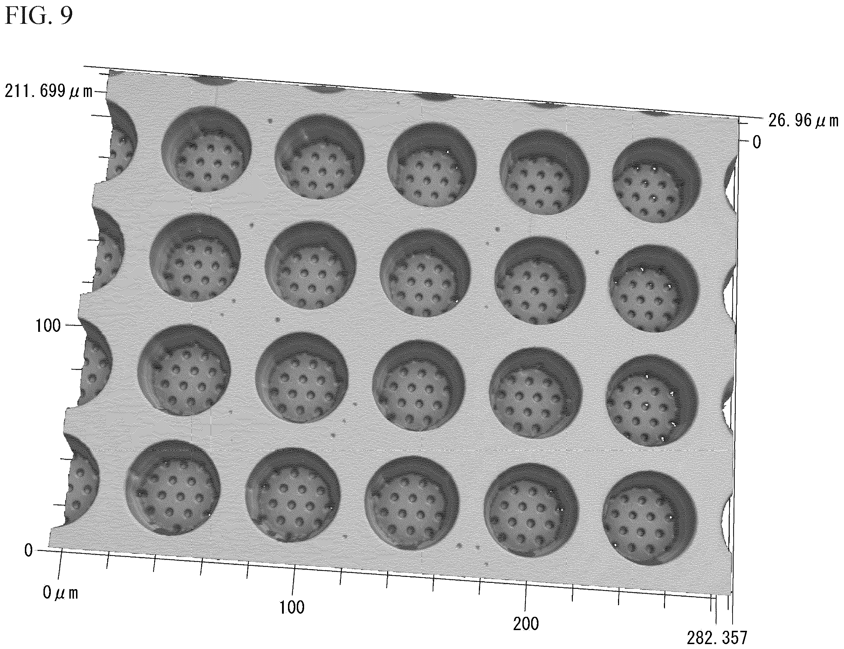

Subsequently, as shown in FIG. 8(b), using a well array-pattern mask, the resist film 710 is exposed by irradiation with active energy rays using an exposure machine. Subsequently, development is performed with a developing solution to remove the portion of the resist film 710 forming the first well 520. As a result, a well array 600 having a first well 520 and a second well 530 is obtained. FIG. 9 is a photomicrograph of a well array having a first well and a second well, actually manufactured by the inventors.

In the method of the present aspect, it is preferable that 0 or 1 of the target nucleic acid fragment is introduced per reaction space. Digital measurement can be performed by introducing 0 or 1 of the target nucleic acid fragment per reaction space. That is, the number of wells in which fluorescent light is detected can be made to correspond to the number of molecules of the target nucleic acid fragment in the sample.

### (Sealing liquid)

The sealing liquid is preferably immiscible with water. "Immiscible with water" means that in a case where water and the sealing liquid are sufficiently mixed and then allowed to stand still, the mixture is separated into an aqueous phase and an organic phase. In addition, it is preferable that the sealing liquid has low water absorption. Low water absorption means that a volume change of an organic layer is 1% or less in a case of being mixed with an equal capacity of water at 20°C and allowed to stand still and being separated into an aqueous phase and an organic phase.

As the sealing liquid, a substance that has a boiling point of about 100°C or higher and is liquid at room temperature can be used. Examples of a specific sealing liquid include fluorine-based liquid such as FC-40, FC-43, FC-770, FC-72, and FC-3283 (all manufactured by 3M) and Fomblin (registered trademark) Oil (Solvay Corporation), mineral oil (Sigma-Aldrich), linear or branched saturated or unsaturated hydrocarbon having 7 to 17 carbon atoms, and the like. One type of these may be used alone, or two or more types may be mixed and used.

Examples of the linear or branched saturated or unsaturated hydrocarbon having 7 to 17 carbon atoms include heptane (C₇ H₁₆), octane (C₈H₁₈), nonane (C₉H₂₀), decane (C₁₀H₂₂), undecane (C₁₁H₂₄), dodecane (C₁₂H₂₆), tridecane (C₁₃H₂₈), tetradecane (C₁₄H₃₀), pentadecane (C₁₅H₃₂), hexadecane (C₁₆H₃₄), heptadecane (C₁₇H₃₆), heptene (C₇H₁₄), octene (C₈H₁₆), nonene (C₉H₁₈), decene (C₁₀H₂₀), undecene (C₁₁H₂₂), dodecene (C₁₂H₂₄), tridecene (C₁₃H₂₆), tetradecene (C₁₄H₂₈), pentadecene (C₁₅H₃₀), hexadecene (C₁₆H₃₂), heptadecene (C₁₇H₃₄), and the like. These may be any isomers.

Examples of octane isomers include 1-octane, 2-methylheptane, 3-methylheptane, 2,2-dimethylhexane, 2,3-dimethylhexane, 2,3,3-trimethylpentane, and the like. In addition, examples of isomers of octene include 1-octene, 2-methyl-1-heptene, 2,3-dimethyl-1-hexene, 2-ethyl-1-hexene, 2,3,3-trimethyl-1-butene, and the like.

### (Water-absorbing organic solvent)

As the water-absorbing organic solvent, a water-absorbing organic solvent that has a boiling point of about 100°C or higher, is liquid at room temperature, and is immiscible with water can be used, and examples thereof include a linear or branched saturated or unsaturated aliphatic alcohol having 4 to 11 carbon atoms. "Immiscible with water" means that in a case where water and an organic solvent are sufficiently mixed and then allowed to stand still, the mixture is separated into an aqueous phase and an organic phase. In addition, water absorption means dissolving water. The water-absorbing organic solvent may be a monohydric alcohol, or may be a dihydric or higher alcohol.

Examples of the specific water-absorbing organic solvent include butanol (C₄H₁₀O), pentanol (C₅H₁₂O), hexanol (C₆H₁₄O), heptanol (C₇H₁₆O), octanol (C₈H₁₈O), nonanol (C₉H₂₀O), decanol (C₁₀H₂₂O), undecanol (C₁₁H₂₄O), pentanediol (C₅H₁₂O₂), and the like. These may be any isomers. In addition, one type of these may be used alone, or two or more types may be mixed and used.

For example, examples of isomers of octanol include 1-octanol, isooctyl alcohol, 2-ethylhexanol, and the like. In addition, for example, examples of the isomers of pentanediol include 1,5-pentanediol, 1,2-pentanediol, 2,3-pentanediol, and the like.

The inventors clarified that in a case of using 1-heptanol, 1-octanol, and 1-nonanol, in particular, an increase in fluorescence intensity due to dehydration concentration is acknowledged, and the target substance tends to be detected with high sensitivity.

### (Fluid device)

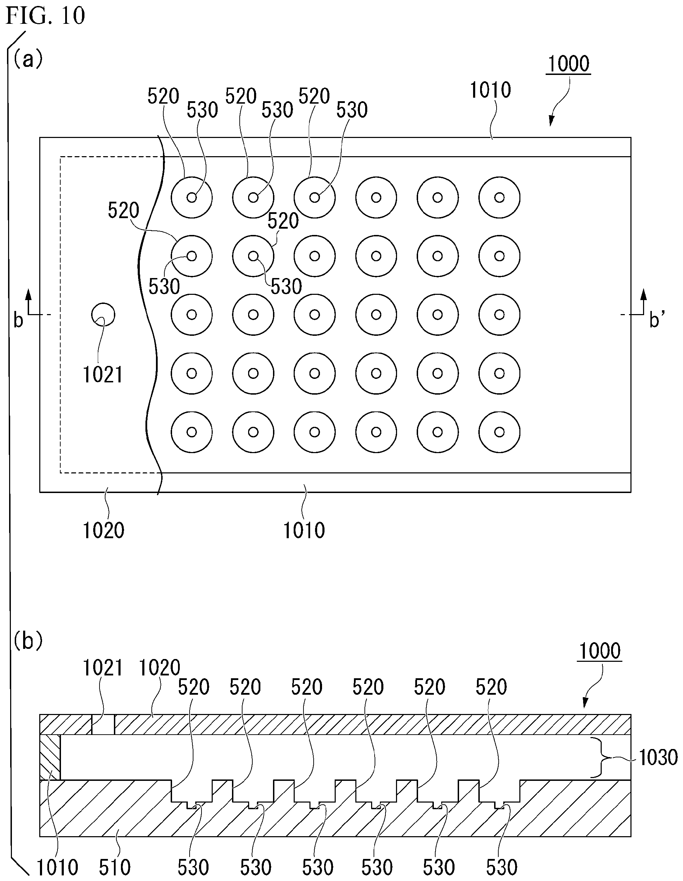

FIG. 10(a) is a top view showing an example of a fluid device including a well array including a plurality of wells having a first well and a second well arranged at a bottom of the first well and having a smaller capacity than the first well; a substrate having a surface on which the well array is arranged; a lid member arranged to face the well array; and a spacer for separating the substrate and the lid member, in which a space between the well array and the lid member forms a flow path through which fluid flows. FIG. 10(b) is a cross-sectional view taken along line b-b' in an arrow direction of FIG. 10(a).

As shown in FIGS. 10(a) and 10(b), a fluid device 1000 includes a well array 500 including a plurality of wells having a first well 520 and a second well 530 arranged at a bottom of the first well 520 and having a smaller capacity than the first well 520, a substrate 510 having a surface on which the well array 500 is arranged, a spacer 1010, and a lid member 1020 having a liquid inlet 1021 formed thereon. A space 1030 between the substrate 510 and the lid member 1020 functions as a flow path through which a sample, a detection reagent, a sealing liquid, a water-absorbing organic solvent, and the like flow.

### (Method for detecting target nucleic acid fragment-first aspect)

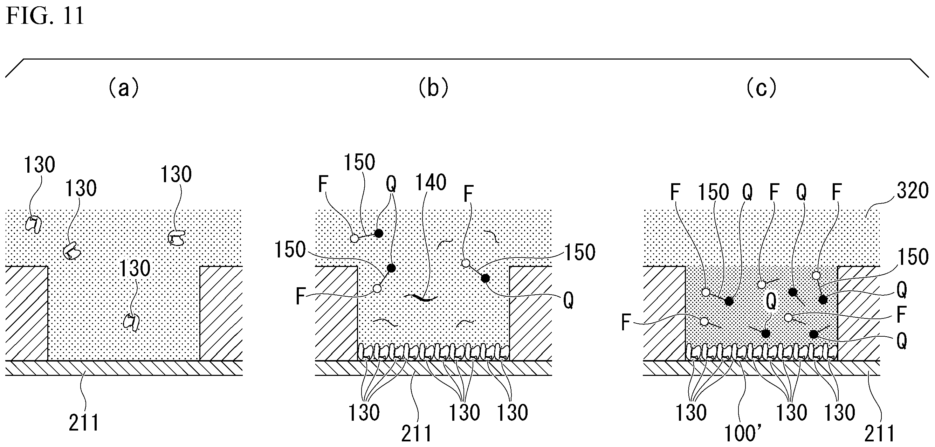

Here, the method for detecting a target nucleic acid fragment according to the first aspect will be described more specifically with reference to FIGS. 11(a) to 11(c). FIGS. 11(a) to 11(c) are schematic cross-sectional views showing an example of procedures for carrying out a method of detecting a target nucleic acid fragment using the fluid device 200 shown in FIG. 2.

In FIGS. 11(a) to 11(c), single-stranded RNA fragments (tgRNA) are detected as target nucleic acid fragments. In addition, Cas13a protein is used as a CRISPR/Cas family protein. In addition, crRNA is used as gRNA.

First, the two-part complex 130 of Cas13a protein and crRNA is immobilized on the inner surface of each well 211 of the well array 212. As shown in FIG. 11(a), the two-part complex 130 is introduced through the liquid inlet 231 of the fluid device 200.

As a result, as shown in FIG. 11(a), the inside of the well 211 and the space between the substrate 210 and the lid member 230 are filled with the two-part complex 130 (Cas13a-crRNA). Although not shown in FIG. 11, the Cas13a protein is biotinylated. In addition, the inner surface of the well 211 (here, only the bottom surface) is biotinylated and further bound with avidin. Therefore, as shown in FIG. 11(b), the two-part complex introduced into the well 211 is immobilized on the bottom surface of the well 211.

Subsequently, the sample and substrate nucleic acid fragment 150 are introduced through the liquid inlet 231 of the fluid device 200. As a result, as shown in FIG. 11(b), the target nucleic acid fragment 140 (tgRNA) in the sample is introduced into the well 211. In addition, as shown in FIG. 11(c), the target nucleic acid fragment 140 in the sample binds to the two-part complex to form a three-part complex 100' (Cas13a-crRNA-tgRNA).

Subsequently, as shown in FIG. 11(c), each well of the well array is sealed with a sealing liquid. Specifically, a sealant 320 is introduced through the liquid inlet 231. In a case where the sealant 320 is introduced, the opening portion of the well 211 is sealed with the sealant 320 in a state in which the inside of the well 211 is filled with the three-part complex 100' and the substrate nucleic acid fragment 150. As a result, each well forms an independent reaction space.

Subsequently, the substrate nucleic acid fragment 150 is cleaved by the nuclease activity of the three-part complex 100', and the fluorescent substance F is separated from the quencher Q. As a result, in a case where the well into which the target nucleic acid fragment 140 has been introduced is irradiated with excitation light, fluorescent light is generated.

As will be described later in examples, as Cas13a is immobilized on a solid phase, the formation efficiency of the three-part complex is significantly improved compared to a case where Cas13a is not immobilized on a solid phase. That is, the detection sensitivity of the target nucleic acid fragment is significantly improved.

### (Method for detecting target nucleic acid fragment-second aspect)

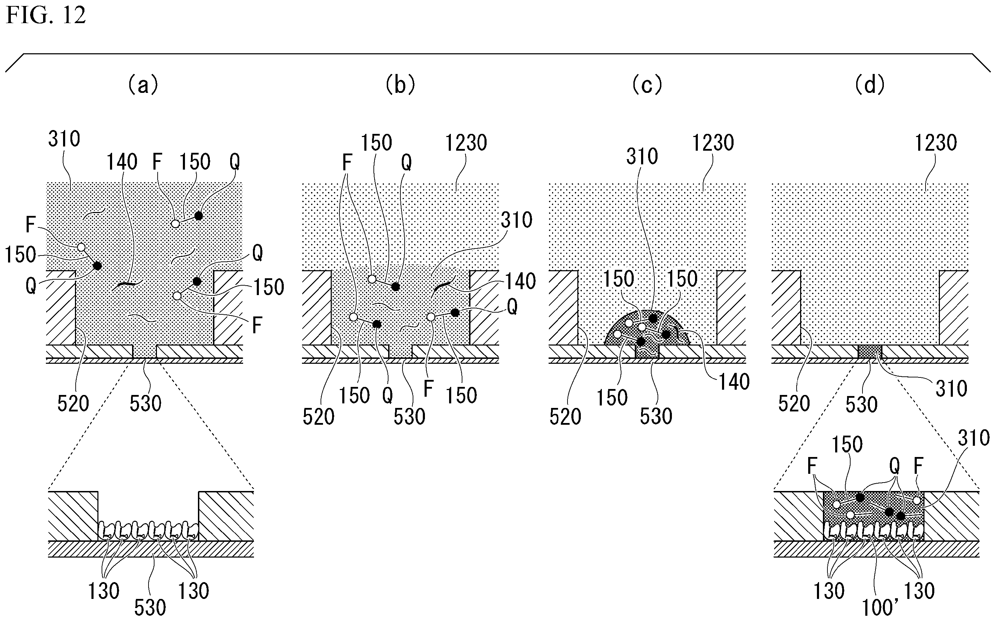

Subsequently, the method for detecting a target nucleic acid fragment according to the second aspect will be described in more detail with reference to FIGS. 12(a) to 12(d). The detection method of the present aspect is mainly different from the detection method of the first aspect described with reference to FIG. 11 in that each well of the well array has a first well 520 and a second well 530, and concentration of the assay solution is performed using a water-absorbing organic solvent.

FIGS. 12(a) to 12(d) are schematic cross-sectional views showing an example of procedures for carrying out a method for detecting a target nucleic acid fragment using the fluid device 1000 shown in FIG. 10. In FIGS. 12(a) to 12(d), a single-stranded RNA fragment (tgRNA) is detected as a target nucleic acid fragment. In addition, Cas13a protein is used as a CRISPR/Cas family protein. In addition, crRNA is used as gRNA.

In FIG. 12(a), the two-part complex 130 of Cas13a protein and crRNA is immobilized on the inner surface (here, only the bottom surface) of the second well 530.

In the example of FIG. 12(a), since the Cas13a protein has formed a gRNA and a two-part complex 130 in advance, as shown in FIG. 12(a), the assay solution 310 containing a sample and the substrate nucleic acid fragment 150 is introduced through the liquid inlet 1021 of the fluid device 1000. As a result, as shown in FIG. 12(a), the inside of the first well 520, the inside of the second well 530, and the space 1030 between the substrate 510 and the lid member 1020 are filled with the assay solution 310.

In a case where the Cas13a protein has not formed the two-part complex 130 with gRNA in advance, an assay solution 310' containing the sample, crRNA and the substrate nucleic acid fragment 150 may be introduced through the liquid inlet 1021 of the fluid device 1000.

Subsequently, each well of the well array is sealed with a sealant 320 (not shown). Specifically, a sealant 320 is introduced through the liquid inlet 1021. In a case where the sealant 320 is introduced, an opening portion of the well 520 is sealed with the sealant 320 in a state in which the inside of the first well 520 is filled with the assay solution 310. As a result, each well forms an independent reaction space.

Subsequently, as shown in FIG. 12(b), the sealant 320 is replaced with a water-absorbing organic solvent 1230. As a result, a content of the well (assay solution 310) is dehydrated, a volume is reduced (concentrated), a Cas 13a-crRNA-tgRNA three-part complex 100' in the assay solution 310 is formed, and the substrate nucleic acid fragment 150 is cleaved. As a result, a fluorescent substance F binding to the substrate nucleic acid fragment 150 is separated from a quencher Q, and fluorescent light is generated by irradiation with excitation light. Detecting fluorescent light in a well indicates that a target substance is present in the well.

In a case where the target nucleic acid fragment 140 is not present in the assay solution 310, the three-part complex 100' is not formed, and thus the substrate nucleic acid fragment 150 is not cleaved and fluorescent light is not generated.

In addition, in a case where an amount of dehydration reaches a desired amount, the water-absorbing organic solvent 1230 may be replaced with the sealant 320 again. With this, it is possible to stop dehydration of the content of the well (assay solution 310). That is, the detection method of the present aspect may further include a step of replacing the water-absorbing organic solvent 1230 with the sealant 320.

In this way, by capturing the target nucleic acid fragment 140 in the large-capacity first well 520, it is possible to improve the capture probability of the target nucleic acid fragment 140, and by performing detection in the small-capacity second well 530, it is possible to detect the target nucleic acid fragment 140 with high sensitivity, and the time required for detection is also shortened.

### [Kit for detecting target nucleic acid fragment]

In one aspect, the present description provides a kit for detecting a target nucleic acid fragment including a substrate on which wells having a volume of 10 aL to 100 pL are formed, a gRNA complementary to the target nucleic acid fragment, a CRISPR/Cas family protein immobilized on a solid phase, and a substrate nucleic acid fragment.

In the kit of the present aspect, the CRISPR/Cas family protein expresses nuclease activity after forming a three-part complex with the gRNA and the target nucleic acid fragment, the substrate nucleic acid fragment is labeled with a fluorescent substance and a quencher, and in a case where the fluorescent substance cleaved by nuclease activity of the three-part complex is separated from the quencher, fluorescent light is emitted by irradiation with excitation light.

By using the kit of the present aspect, it is possible to suitably carry out the above-described method of detecting a target nucleic acid fragment. In the kit of the present aspect, the substrate with wells formed on the surface, the target nucleic acid fragment, the gRNA, the CRISPR/Cas family protein, and the substrate nucleic acid fragment are the same as those described above.

In the kit of the present aspect, the CRISPR/Cas family protein may be immobilized on the inner surface of the well. In addition, the well has a first well and a second well arranged at a bottom of a first well and having a smaller capacity than the first well, and the CRISPR/Cas family protein may be immobilized on the inner surface of the second well.

Here, the method of binding the CRISPR/Cas family protein to the inner surface of the well is the same as described above. In addition, the well having the first well and the second well is also the same as described above.

Alternatively, the CRISPR/Cas family protein may be immobilized on the surface of the particle. Here, the method of binding the CRISPR/Cas family protein to the particle and the surface of the particle is the same as described above.

In the kit of the present aspect, the CRISPR/Cas family protein may be a Cas12 protein or a Cas13 protein. The specific Cas12 protein or Cas13 protein is the same as described above.

### [Examples]

Subsequently, the present invention will be described in more detail with reference to examples, but the present invention is not limited to the following examples.

### [Material and method]

### (Preparation of Cas13a protein)

An expression vector for Leptotrichia wadei Cas13a (LwCas13a) was transfected into Escherichia coli BL21 (DE3) strain for expression. The expression vector was a pET-based vector with a 10 x His tag, maltose binding protein (MBP), and TEV protease cleavage site at the N-terminus. The expressed Cas13a protein was purified using Ni-NTA resin. Subsequently, after reacting TEV protease at 4°C overnight, cation exchange chromatography was performed using MBPTrap HP column (GE Healthcare) and HiTrap Heparin HP column (GE Healthcare) connected thereto, and purification was further performed by gel filtration chromatography using a Superdex 200 column (GE Healthcare).

### (Preparation of target nucleic acid fragment)

As the target nucleic acid fragment, a single-stranded RNA fragment (SEQ ID NO: 5) chemically synthesized by outsourcing (IDT), or a full-length SARS-CoV2 N gene RNA (SEQ ID NO: 9) synthesized by in vitro transcription (IVT) was used.

### (Preparation of gRNA)

A DNA fragment encoding gRNA (crRNA) was prepared by PCR amplification using an overlapping primer containing a T7 promoter sequence, a 20 base target sequence and a scaffold sequence as a template. Subsequently, the obtained DNA fragment was subjected to an in vitro transcription reaction to prepare crRNA. Base sequences of the used crRNAs are shown in SEQ ID NO: 4 and SEQ ID NO: 8.

### (Preparation of substrate nucleic acid fragment)

A substrate nucleic acid fragment (single-stranded RNA fragment) was chemically synthesized by outsourcing (IDT). A 5' end of the substrate nucleic acid fragment was labeled with FAM, which is a fluorescent substance, and a 3' end was labeled with Iowa Black FQ (IDT), which is a quencher. The base sequence of the chemically synthesized substrate nucleic acid fragment (single-stranded RNA fragment) was "5'-(FAM)UUUUU(IABkFQ)-3'" (here, "IABkFQ" stands for Iowa Black FQ).

### (Preparation of well array A)

A well array A was prepared by the same procedure as in FIGS. 7(a) to 7(f), described above. First, as shown in FIG. 7(a), a glass substrate 510 was immersed in an 8 M potassium hydroxide solution for about 24 hours to form a hydroxyl group on a surface.

Subsequently, as shown in FIG. 7(b), the surface of the glass substrate 510 was spin-coated with a fluorine resin (CYTOP, manufactured by AGC Inc.) to form a film 700. A condition of the spin coating was 1,000 rpm (revolutions per minute) for 30 seconds. Under this condition, a film thickness of the film 700 is about 1.8 µm.

Subsequently, the film 700 was adhered to the surface of the glass substrate 510 by performing baking on a hot plate at 180°C for 1 hour to cause dehydration condensation between a silanol group of the film 700 (CYTOP) and a hydroxyl group on the glass surface.

Subsequently, as shown in FIG. 7(c), a resist (product name "AZ-P4903", manufactured by AZ Electronic Materials) was spin-coated on the surface of the film 700 at 4000 rps for 60 seconds to form a resist film 710.

Subsequently, the resist film 710 was adhered to the surface of the film 700 by baking the glass substrate 710 on a hot plate at 110°C for 1 hour to evaporate an organic solvent in the resist film 710.

Subsequently, as shown in FIG. 7(d), using a well array-pattern mask, the resist film 710 was exposed by irradiation with ultraviolet rays at 250 W for 14 seconds using an exposure machine (manufactured by Union Optical Co., Ltd.). Subsequently, the resist film 710 was developed by being immersed in a developing solution (AZ developer, manufactured by AZ Electronic Materials) for 1.5 minutes. As a result, a portion of the resist film 710 forming the well was removed.

Subsequently, as shown in FIG. 7(e), the film 700 masked with the resist film 710 was dry-etched under conditions of 200 sccm of O₂, a pressure of 5 Pa, and an output of 50 W for 30 minutes using a Reactice ion etching device (manufactured by YAC) to form a well 530 on the film 700.

Subsequently, as shown in FIG. 7(f), the glass substrate 510 was immersed in acetone, washed with isopropanol, and then washed with pure water to remove the resist film 710 and obtain an array of the well 530 (well array A). The well array A had a shape in which 1,500,000 cylindrical wells 530 with a diameter of 3.5 µm and a depth of 1.8 µm were arranged in an area of 1 cm². The volume per well of the well 530 was 17 fL.

### (Preparation of well array B)

A well array B having a first well and a second well was prepared by the same procedures as those shown in FIGS. 7(a) to 7(f), 8(a), and 8(b). First, the well array obtained in the same manner as in FIGS. 7(a) to 7(f) was dry-etched (13 sccm of O₂, a pressure of 14 Pa, an output of 125 W) for 5 seconds using a Reactice ion etching device (manufactured by Samco) to perform hydrophilic treatment on a well array surface. Subsequently, the well array was placed on a hot plate at 65°C, and a sheet-type resist (product name "SU-8 3020CF DFR Type-S", manufactured by KAYAKU Advanced Materials, Inc.) was adhered using a laminate roller to form a resist film 710, as shown in FIG. 8(a).

Subsequently, as shown in FIG. 8(b), using a well array-pattern mask, the resist film 710 was exposed by irradiation with ultraviolet rays for 20 seconds using an exposure machine (manufactured by Union Optical Co., Ltd.). Subsequently, the resist film 710 was developed by being immersed in a developing solution (product name: "SU8 developer", manufactured by KAYAKU Advanced Materials, Inc.) for 8 minutes. As a result, a portion of the resist film 710 forming the well was removed to obtain a well array B having the first well 520 and the second well 530.

The well array B had a shape in which a well array obtained by 40,000 cylindrical wells 520 with a diameter of 40 µm and a depth of 20 µm being arranged in 1 cm² was stacked on a well array obtained by 1,500,000 cylindrical wells 530 with a diameter of 3.5 µm and a depth of 1.8 µm being arranged in 1 cm².

The well array B had a shape in which 12 to 18 wells 530 were arranged at a bottom of each well 520. FIG. 9 is a photomicrograph of the prepared well array B.

### (Preparation of fluid device A)

In the same manner as in FIG. 2, a spacer 220 was arranged in the above-described well array A, and a glass plate 230 having a liquid inlet 231 formed thereon was placed to prepare a fluid device A. As a result, a fluid device A was obtained in which a space between the well array A and the glass plate 230 was a flow path.

### (Preparation of fluid device B)

In the same manner as in FIG. 10, a spacer 1010 was arranged on the above-described well array B, and a glass plate 1020 having a liquid inlet 1021 formed thereon was placed to prepare a fluid device B. As a result, a fluid device B was obtained in which the space between the well array B and the glass plate 1020 was a flow path.

### [Experimental Example 1]

### (Examination using Cas13a)

a Cas13a protein, a gRNA (SEQ ID NO: 4), and a target nucleic acid fragment were mixed in a buffer A having a composition shown in Table 1 below so that a final concentration of the Cas13a protein is 40 nM, a final concentration of the gRNA is 25 nM, and a final concentration of the target nucleic acid fragment is 30 pM, 3 pM, 0.3 pM, or 0 pM, thereby forming a three-part complex. Hereinafter, the solution is referred to as a three-part complex solution.

**[Table 1]**

| | |
|---|---|
| Buffer A | |
| 20 mM HEPES (pH 7.5), 150 mM KCl | 19.89 mL |
| 2 M MgCl₂ | 100 µL |
| 1 M DTT | 10 µL |
| Total | 20 mL |

Four of the above-described fluid devices A were prepared. In addition, a solution was prepared by dissolving the substrate nucleic acid fragment in the buffer A to a final concentration is 10 µM.

Subsequently, each of the above-described three-part complex solution and an assay solution in which the substrate nucleic acid fragment was mixed was prepared, and immediately introduced through the liquid inlet of each fluid device A. As a result, the assay solution was introduced into each well of the well array.

Subsequently, a sealant (hexadecane, Sigma-Aldrich) was introduced through the liquid inlet of each fluid device A. As a result, the well into which the assay solution was introduced was sealed with a sealant, and each well became each independent reaction space. After several minutes, the well array of each fluid device A was observed under a fluorescence microscope.

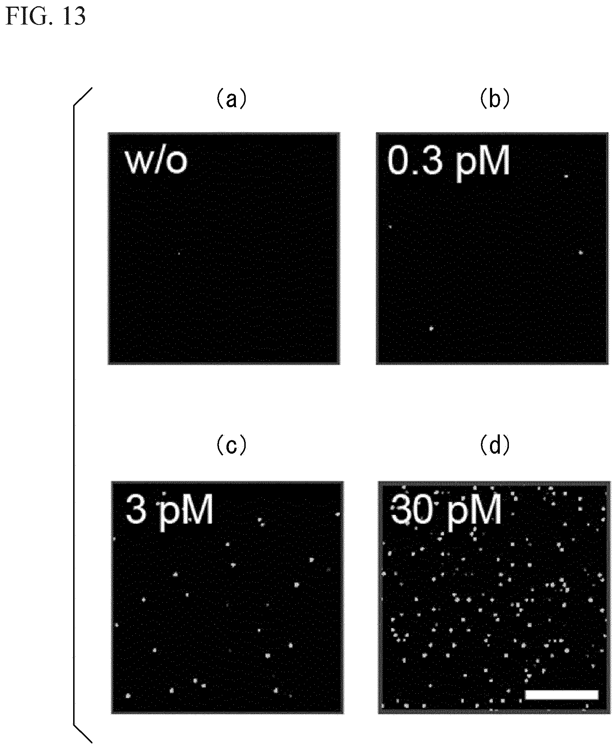

FIG. 13(a) is a representative fluorescence micrograph showing results of an assay solution in which a final concentration of the target nucleic acid fragment is 0 pM. FIG. 13(b) is a representative fluorescence micrograph showing results of an assay solution in which a final concentration of the target nucleic acid fragment is 0.3 pM. FIG. 13(c) is a representative fluorescence micrograph showing results of an assay solution in which a final concentration of the target nucleic acid fragment is 3 pM. In addition, FIG. 13(d) is a representative fluorescence micrograph showing results of an assay solution in which a final concentration of the target nucleic acid fragment is 30 pM. Scale bar is 50 µm.

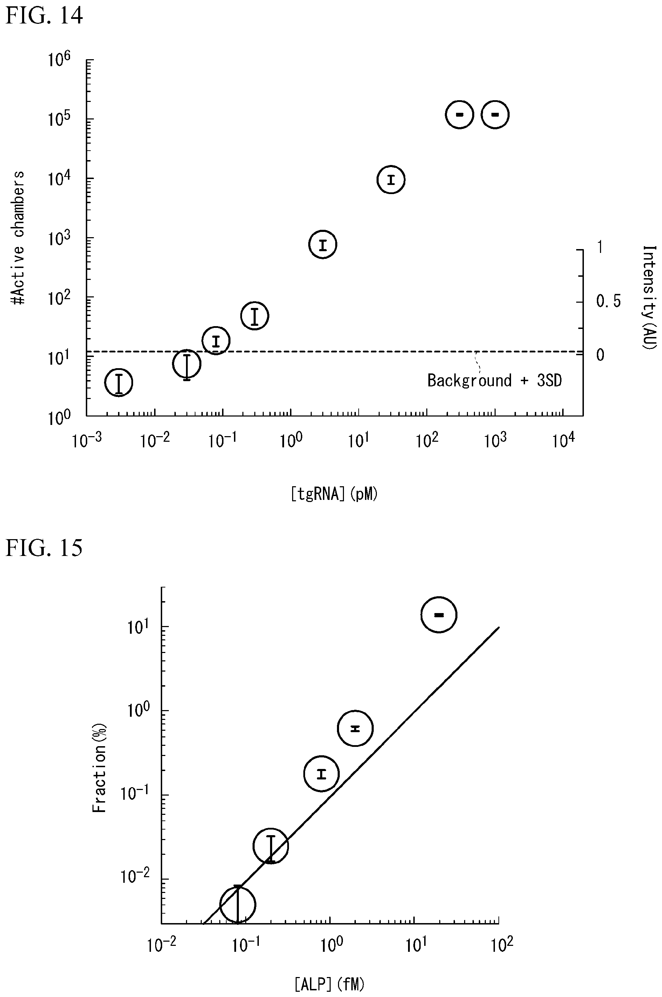

FIG. 14 is a graph showing a relationship between the number of wells in which fluorescent light was detected and the final concentration of the target nucleic acid fragment. The vertical axis indicates the number of wells in which fluorescent light was detected, and the horizontal axis indicates the final concentration of the target nucleic acid fragment. As a result, it became clear that the detection sensitivity was about 50 fM.

### [Experimental Example 2]

### (Concentration examination)

An assay solution was prepared by mixing serially diluted alkaline phosphatase (Sigma-Aldrich) with 1 µM of fluorescent substrate (sTG-phos), and introduced through the liquid inlet of the above-described fluid device B. As a result, the assay solution was introduced into each well of the well array. Chemical formula of sTG-phos is shown in Formula (1) below (refer to Sakamoto S., et al., Multiplexed single-molecule enzyme activity analysis for counting disease-related proteins in biological samples, Sci Adv. 6 (11), eaay0888, 2020.).

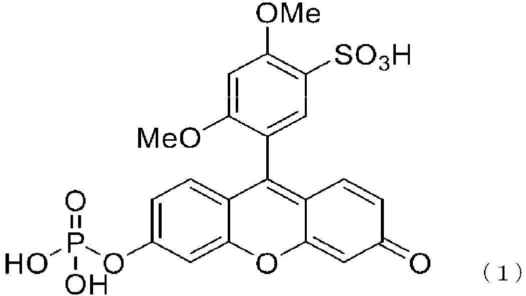

Subsequently, a sealant (hexadecane, Sigma-Aldrich) was introduced through the liquid inlet of the fluid device B. As a result, the well into which the assay solution was introduced was sealed with a sealant, and each well became each independent reaction space.

Subsequently, 1-octanol was introduced through the liquid inlet of the fluid device to replace the sealant. As a result, a content of the well is dehydrated, a volume is reduced, and alkaline phosphatase and sTG-phos in the content are reacted with each other to generate a fluorescent substance (sTG). The chemical formula of sTG is shown in Formula (2) below.

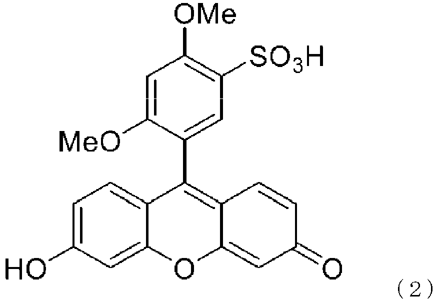

Subsequently, 2.5 minutes after the introduction of 1-octanol, 1-octanol was replaced with a sealant (product name "Fomblin (registered trademark) Oil", Solvay Corporation). As a result, the dehydration of the content of the wells was stopped. Subsequently, sTG fluorescent light was detected. FIG. 15 is a representative graph showing a proportion (%) of wells exhibiting predetermined fluorescence intensity (relative value) based on a photograph of the well array in which the sTG fluorescent light was detected. In FIG. 15, the horizontal axis of the graph indicates the concentration of alkaline phosphatase (ALP).

As a result, it was clarified that the presence of about 80 aM of alkaline phosphatase could be detected. That is, it was clarified that the detection sensitivity in a case of performing dehydration concentration using 1-octanol, using the fluid device B, was about 80 aM.

### [Experimental Example 3]

### (Immobilization of Cas13a)

Four of the above-described fluid devices A were prepared. Subsequently, an inner surface of a well 530 of the fluid device A was biotinylated.

Biotinylation was performed as follows. First, a thiol group was modified on a glass surface of a bottom surface of the well 530 of the fluid device A by silane coupling treatment using (3-mercaptopropyl)-trimethoxysilane. Subsequently, biotin bound to the above thiol group by maleimide reaction using biotin-dPEG₁₁-maleimide. Through the above operation, the inner surface of the well 530 of the fluid device A was biotinylated.

Subsequently, avidin was suspended in a buffer A having the composition shown in Table 1 above so that a final concentration was 1 mg/mL, and introduced through the liquid inlet of the fluid device A. As a result, avidin was introduced into each well of the well array and bound to biotin bound to the inner surface of the well 530.

On the other hand, a lysine residue of the Cas13a protein or the N-terminus of the Cas13a protein was modified with NHS-PEG₄-biotin and biotinylated. In addition, a cysteine residue of the biotinylated Cas13a protein was also labeled with Alexa488-maleimide. Subsequently, the biotinylated Cas13a protein was introduced through the liquid inlet of the fluid device A. As a result, the biotinylated Cas13a protein was introduced into each well of the well array, bound to avidin bound to the inner surface of the well 530, and immobilized. Subsequently, Alexa647 was dissolved in water, introduced through the liquid inlet of the fluid device A, and observed with a fluorescence microscope.

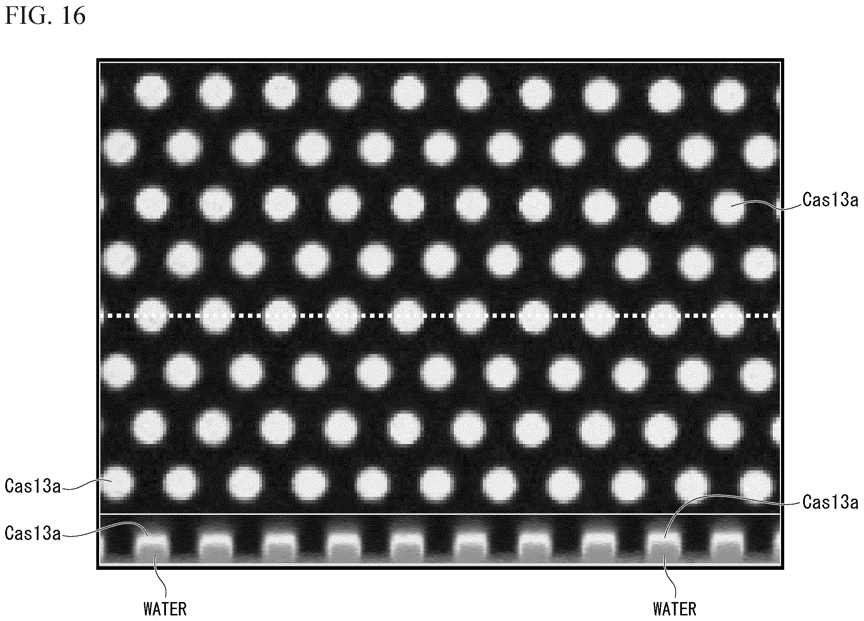

FIG. 16 is a fluorescence micrograph of the fluid device A. FIG. 16 is a photograph of the fluid device A taken from above, and a bottom of FIG. 16 is a cross-sectional photograph of the fluid device A taken along a dotted line in FIG. 16. The photograph in the bottom of FIG. 16 is upside down, and an upper side of the photograph is the bottom surface of the fluid device A. In FIG. 16, Alexa488 fluorescent light indicates the presence position of the Cas13a protein. In addition, Alexa647 fluorescent light indicates the presence position of water introduced into the well. As a result, it was clarified that the Cas13a protein could be immobilized only on the inner surface of the well 530.

### [Experimental Example 4]

### (Examination 1 using immobilized Cas13a)

The Cas13a protein immobilized on the inner surface of the well was used to detect a target nucleic acid fragment. First, the lysine residue of the Cas13a protein or the N-terminus of the Cas13a protein was modified with NHS-PEG4-biotin and biotinylated. Subsequently, the biotinylated Cas13a protein and gRNA (SEQ ID NO: 4) were mixed with the buffer A having the composition shown in Table 1 so that the final concentration of the biotinylated Cas13a protein is 40 nM and the final concentration of gRNA is 25 nM, thereby forming a two-part complex.

Subsequently, in the same manner as in Experimental Example 3, four of the fluid devices A each having a two-part complex immobilized on the inner surface of each well of the fluid device A were prepared.

In addition, a solution dissolved in the buffer A having the composition shown in Table 1 below was prepared so that the final concentration of the substrate nucleic acid fragment is 10 µM, the final concentration of the target nucleic acid fragment (SEQ ID NO: 5) is 3 pM, 0.3 pM, 30 fM, or 3 fM, in the buffer A, and used as an assay solution.

Subsequently, each assay solution was introduced through the liquid inlet of each fluid device A. As a result, the assay solution was introduced into each well of the well array.

Subsequently, a sealant (hexadecane, Sigma-Aldrich) was introduced through the liquid inlet of each fluid device A. As a result, the well into which the assay solution was introduced was sealed with a sealant, and each well became each independent reaction space. After several minutes, the well array of each fluid device A was observed under a fluorescence microscope.

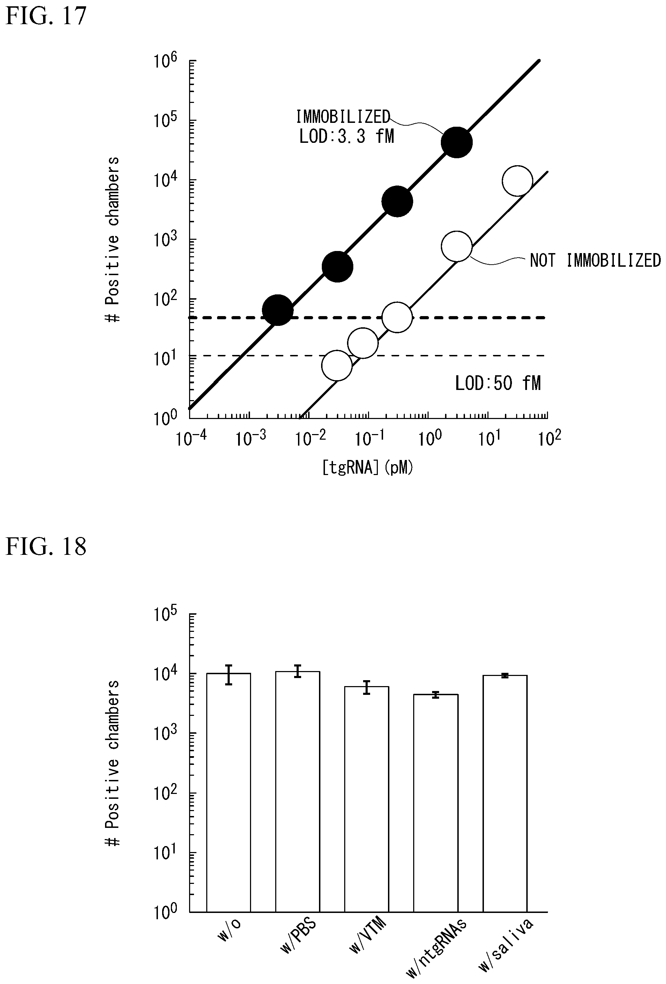

FIG. 17 is a graph showing a relationship between the number of wells in which fluorescent light was detected and the final concentration of the target nucleic acid fragment (tgRNA). The vertical axis indicates the number of wells in which fluorescent light was detected, and the horizontal axis indicates the final concentration of the target nucleic acid fragment. As a result, it was clarified that the detection sensitivity in a case of immobilizing Cas13a protein on the inner surface of the well was about 3.3 fM. For comparison, FIG. 17 also shows the results in a case where the two-part complex was not immobilized, measured in Experimental Example 1.

As a result, it was clarified that the detection sensitivity was significantly improved from about 50 fM to about 3.3 fM by immobilization of the Cas13a protein.

### [Experimental Example 5]

### (Examination 1 of influence of contaminants)

The influence of contaminants in detection of a target nucleic acid fragment was examined. First, the lysine residue of the Cas13a protein or the N-terminus of the Cas13a protein was modified with NHS-PEG4-biotin and biotinylated. Subsequently, the biotinylated Cas13a protein and gRNA (SEQ ID NO: 4) were mixed with the buffer A having the composition shown in Table 1 so that the final concentration of the biotinylated Cas13a protein is 40 nM and the final concentration of gRNA is 25 nM, thereby forming a two-part complex.

Subsequently, in the same manner as in Experimental Example 3, a fluid device A in which the two-part complex was immobilized on the inner surface of each well of the fluid device A was prepared. Five similar fluid devices A were prepared.

In addition, an assay solution was prepared by adding the substrate nucleic acid fragment to a final concentration of 10 µM, adding the target nucleic acid fragment to a final concentration of 30 pM, and further adding contaminants, to the buffer A.

As contaminants, based on the assay solution, a 10 v/v% phosphate buffered saline (PBS), a 70 v/v% virus transport solution (VTM, catalog number "SGVTM-3R", Sugiyama-Gen Co., Ltd.), a non-target nucleic acid fragment having a final concentration 3 ng/µL, and a 10 v/v% saliva were used. In addition, since saliva contains RNase, 1 mM Triton X-100, which is a surfactant, was added in advance and then heated at 90°C for 5 minutes to deactivate RNase. In addition, an assay solution in which contaminants were not added was also prepared for comparison.

Subsequently, each assay solution was introduced through the liquid inlet of each fluid device A. As a result, the assay solution was introduced into each well of the well array.

Subsequently, a sealant (hexadecane, Sigma-Aldrich) was introduced through the liquid inlet of each fluid device A. As a result, the well into which the assay solution was introduced was sealed with a sealant, and each well became each independent reaction space. After several minutes, the well array of each fluid device A was observed under a fluorescence microscope.

FIG. 18 is a graph showing the number of wells in which fluorescent light was detected using each assay solution. The vertical axis indicates the number of wells in which fluorescent light was detected. In FIG. 18, "w/o" is a result of the assay solution to which contaminants were not added, "w/PBS" is a result of the assay solution to which PBS was added, "w/VTM" is a result of the assay solution to which virus transport solution was added, "w/ntgRNAs" is a result of the assay solution to which a non-target nucleic acid fragment was added, and "w/saliva" is a result of the assay solution to which saliva was added.

As a result, it was clarified that even if contaminants were added, almost no change was acknowledged in detection efficiency of the target nucleic acid fragment.

### [Experimental Example 6]

### (Examination 2 of influence of contaminants)

The influence of contaminants in detection of a target nucleic acid fragment was examined. Saliva was used as a contaminant. First, the lysine residue of the Cas13a protein or the N-terminus of the Cas13a protein was modified with NHS-PEG4-biotin and biotinylated. Subsequently, the biotinylated Cas13a protein and gRNA (SEQ ID NO: 4) were mixed with the buffer A having the composition shown in Table 1 so that the final concentration of the biotinylated Cas13a protein is 40 nM and the final concentration of gRNA is 25 nM, thereby forming a two-part complex.

Subsequently, in the same manner as in Experimental Example 3, a fluid device A in which the two-part complex was immobilized on the inner surface of each well of the fluid device A was prepared.

In addition, an assay solution was prepared by adding the substrate nucleic acid fragment to a final concentration of 10 µM, and adding the target nucleic acid fragment (tgRNA) to a final concentration of 30 pM, 3 pM, 300 fM, 80 fM, 30 fM, or 8 fM, to the buffer A.

In addition, an assay solution was prepared by adding the substrate nucleic acid fragment to a final concentration of 5 µM, and adding the target nucleic acid fragment (tgRNA) to a final concentration of 30 pM, 3 pM, 300 fM, or 30 fM, to the saliva. Since saliva contains RNase, 1 mM Triton X-100, which is a surfactant, was added in advance and then heated at 90°C for 5 minutes to deactivate RNase.

Subsequently, each assay solution was introduced through the liquid inlet of each fluid device A. As a result, the assay solution was introduced into each well of the well array.

Subsequently, a sealant (hexadecane, Sigma-Aldrich) was introduced through the liquid inlet of each fluid device A. As a result, the well into which the assay solution was introduced was sealed with a sealant, and each well became each independent reaction space. After several minutes, the well array of each fluid device A was observed under a fluorescence microscope.

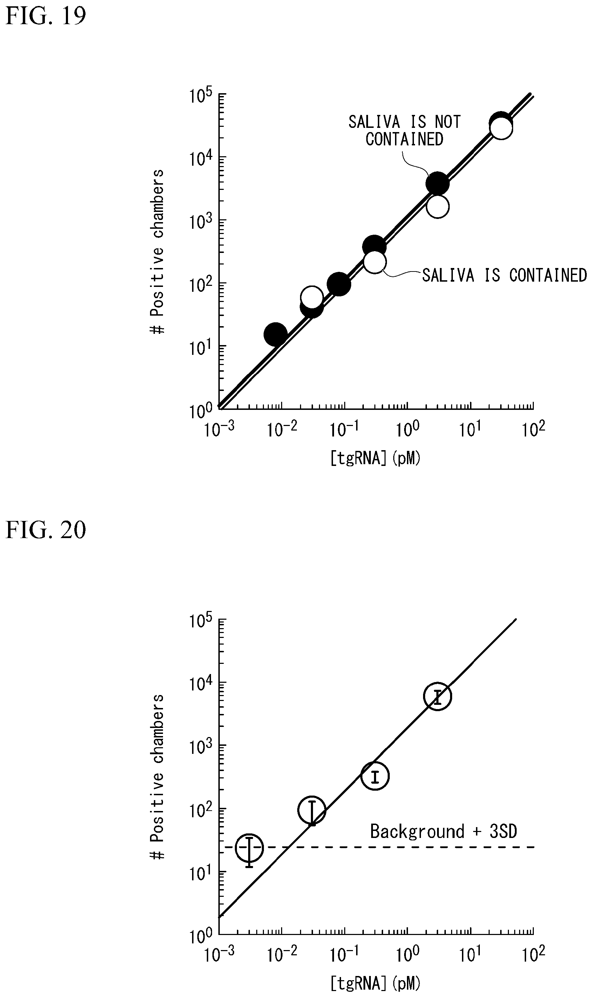

FIG. 19 is a graph showing the relationship between the number of wells in which fluorescent light was detected and the final concentration of the target nucleic acid fragment (tgRNA). The vertical axis indicates the number of wells in which fluorescent light was detected, and the horizontal axis indicates the final concentration of the target nucleic acid fragment. As a result, it was clarified that even if saliva is contained in the assay solution, the target nucleic acid fragment can be detected in the same manner as in a case where saliva is not contained.

This result indicates that purification of the target nucleic acid fragment is unnecessary in a case where saliva is used as a sample. As a result, it was clarified that the detection time for the target nucleic acid can be greatly shortened.

### [Experimental Example 7]

### (Detection of novel coronavirus)

Novel coronavirus (SARS-CoV-2) specimens isolated from the Diamond Princess were used to detect novel coronavirus in a sample. SARS-CoV-2 virus was grown in VeroE6/TMPRSS2 cells and purified using the RNeasy Mini kit (Qiagen).

In addition, the Cas13a protein and the gRNA (SEQ ID NO: 6) were mixed with the buffer A having the composition shown in Table 1 below so that the final concentration of the Cas13a protein was 40 nM and the final concentration of the gRNA was 25 nM, thereby forming the two-part complex.

In addition, the assay solution was prepared by adding the substrate nucleic acid fragment to a final concentration of 10 µM, and adding the novel coronavirus to a final concentration of 3 pM, 0.3 pM, 0.03 pM, 0.003 pM, or 0 pM, to the buffer A.

Subsequently, five of the above-described fluid devices A were prepared, and each assay solution was introduced through the liquid inlet of each fluid device A. As a result, the assay solution was introduced into each well of the well array.

Subsequently, a sealant (hexadecane, Sigma-Aldrich) was introduced through the liquid inlet of each fluid device A. As a result, the well into which the assay solution was introduced was sealed with a sealant, and each well became each independent reaction space. After several minutes, the well array of each fluid device A was observed under a fluorescence microscope.

FIG. 20 is a graph showing the relationship between the number of wells in which fluorescent light was detected and the final concentration of novel coronavirus. The vertical axis indicates the number of wells in which fluorescent light was detected, and the horizontal axis indicates the final concentration of novel coronavirus. As a result, it was demonstrated that the actual virus (SARS-CoV-2) could be detected by the above method. The detection sensitivity was about 16 fM.

### [Experimental Example 8]

### (Examination 2 using immobilized Cas13a)

After hybridizing the target nucleic acid fragment with the single-stranded DNA fragment, particles and the Cas13a protein were mixed to immobilize Cas13a on the surface of the particles, and the target nucleic acid fragment was detected.

First, a single-stranded DNA fragment of which 3' end was modified with biotin (base sequence is shown in SEQ ID NO: 7) and the target nucleic acid fragment (SEQ ID NO: 5) were mixed and incubated at 70°C for hybridization. The single-stranded DNA fragment (SEQ ID NO: 7) had a base sequence complementary to a part of the target nucleic acid fragment (SEQ ID NO: 5). Subsequently, a solution of the hybridized single-stranded DNA fragment (SEQ ID NO: 7) and the target nucleic acid fragment (SEQ ID NO: 5) was mixed with streptavidin-coated magnetic beads (product name "Dynabeads M280", Veritas) to obtain a mixture solution.

At this time, the solution was mixed in a buffer B (20 mM HEPES (pH 7.5), 20 mM KCl, 2 mM MgCl₂, 50 µM Triton X-100) so that the final concentration of the single-stranded DNA fragment (SEQ ID NO: 7) was 12 nM and the final concentration of the magnetic beads was 1 mg/mL. In addition, four types of mixture solutions were prepared so that the final concentration of the target nucleic acid fragment (SEQ ID NO: 5) was 0.3 pM, 30 fM, 3 fM, or 0.8 fM.

Subsequently, the buffer B was dispensed into four new plastic tubes, and a Cas13a protein, a gRNA (SEQ ID NO: 4), a substrate nucleic acid fragment, and each of the above mixture solutions were mixed therewith to obtain four types of assay solutions. The final concentration of the substrate nucleic acid fragment was adjusted to be 5 µM.

In addition, a well array C was prepared in which 2,800,000 cylindrical wells with a diameter of 4.0 µm and a depth of 3.0 µm were arranged per 1 cm². The volume per well of the well array C was 50 fL. In addition, the fluid device C was prepared in the same manner as the above-described fluid device A, except that the well array C was used instead of the well array A.

Four fluid devices C were prepared, and each of the assay solutions was introduced through each of the liquid inlet of the fluid devices C and placed on a magnet sheet. As a result, each assay solution was introduced into each well of the well array. In addition, magnetic beads to which the three-part complex containing the target nucleic acid fragment bound were concentrated and captured in each well.

Subsequently, a sealant (mineral oil, Sigma-Aldrich) was introduced through the liquid inlet of each fluid device C. As a result, the wells into which each assay solution and substrate nucleic acid fragment were introduced were sealed with the sealant, and each well became each independent reaction space. After several minutes, the well array of each fluid device C was observed under a fluorescence microscope.

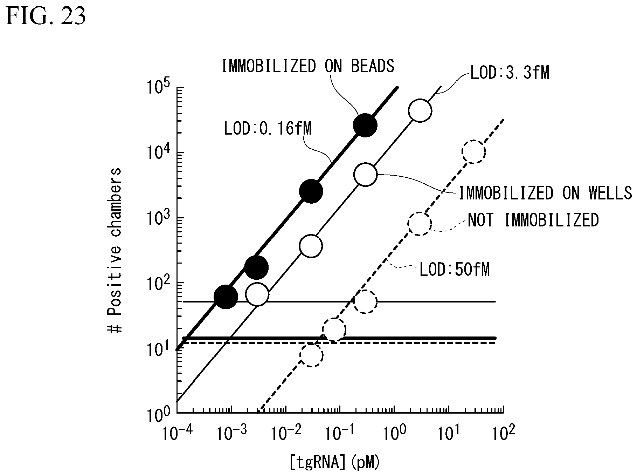

FIG. 23 is a graph showing the relationship between the number of wells in which fluorescent light was detected and the final concentration of the target nucleic acid fragment (tgRNA). The vertical axis indicates the number of wells in which fluorescent light was detected, and the horizontal axis indicates the final concentration of the target nucleic acid fragment. As a result, it was clarified the detection sensitivity was about 0.16 fM. For comparison, FIG. 23 shows the results of a case where the two-part complex was not immobilized, measured in Experimental Example 1, and the results of a case where the two-part complex was immobilized on the inner surface of the well, measured in Experimental Example 4.

As a result, by immobilizing the Cas13a protein on the particles, it was clarified that the detection sensitivity was further significantly improved from about 3.3 fM to about 0.16 fM in a case of being immobilized on the inner surface of the well.

### [Experimental Example 9]

### (Examination 3 using immobilized Cas13a)

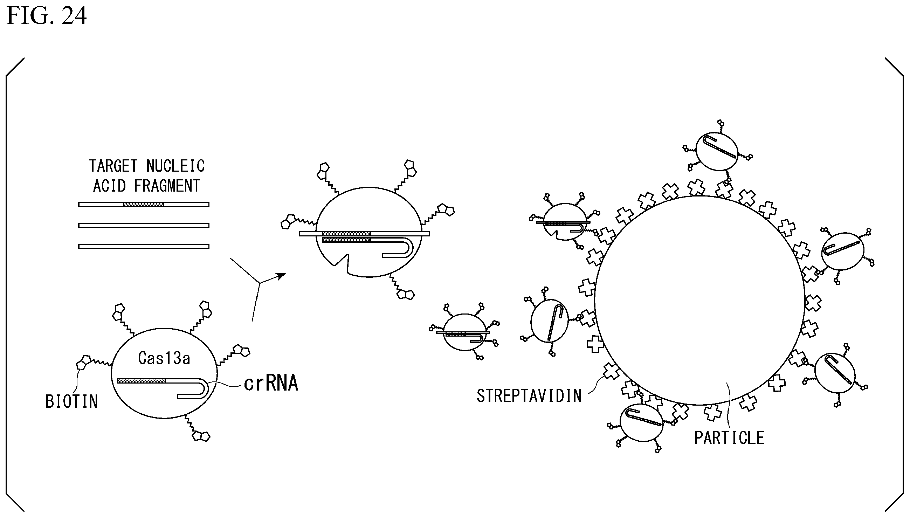

A Cas13a protein immobilized on the surface of the particles was used to detect a target nucleic acid fragment. FIG. 24 is a schematic diagram showing a detection method of the present experimental example. As the particles, streptavidin-coated magnetic beads (product name "Dynabeads MyOne Streptavidin T1", Veritas) were used.

First, the lysine residue of the Cas13a protein or the N-terminus of the Cas13a protein was modified with NHS-PEG4-biotin and biotinylated. Subsequently, the biotinylated Cas13a protein and the gRNA (SEQ ID NO: 8) were mixed in a buffer F (20 mM HEPES-KOH (pH 6.8), 60 mM NaCl, 6 mM MgCl₂, 50 µM Triton X-100) so that the final concentration of the biotinylated Cas13a protein was 3 µM, and the final concentration of the gRNA was 0.75 µM, thereby forming a two-part complex.

Subsequently, the two-part complex was diluted with the buffer F containing the substrate nucleic acid fragment and Alexa647-maleimide to obtain a mixture solution. Here, the concentration of the biotinylated Cas13a protein in the mixture solution was 60 nM, the concentration of the gRNA was 12 nM, the concentration of the substrate nucleic acid fragment was 12 µM, and the concentration of the Alexa647-maleimide was 60 µM.

Subsequently, 20 µL of the above mixture solution was mixed with 100 µL of a buffer E (20 mM HEPES-KOH (pH 7.5), 100 mM KCl, 10 mM MgCl₂, 50 µM Triton X-100) containing the target nucleic acid fragment (SEQ ID NO: 9) so that the final concentration was 300 fM. Subsequently, 20 µL of 0.5 mg/mL magnetic beads were further added and mixed by pipetting for 10 seconds. Subsequently, incubation was performed for 3 minutes to use the solution as an assay solution. Here, for comparison, an assay solution to which magnetic beads were not added was also prepared.

In addition, a well array D was prepared in which 2,000,000 cylindrical wells with a diameter of 3.5 µm and a depth of 3.5 µm were arranged per 1 cm². The volume per well of the well array D was 30 fL. In the present experimental example, the assay solution was dropped directly onto the well array D, and a sealant was further dropped to perform imaging while sealing each well.

Two well arrays D were prepared, and 105 µL of each of the assay solutions was dropped to each of the well arrays D to install a magnet on a bottom of the well array D. As a result, each of the assay solutions was introduced into each well of the well array D. In addition, in a case where the assay solution contained magnetic beads, the magnetic beads bound with the three-part complex containing the target nucleic acid fragment were concentrated and captured in each well.

Subsequently, 95 µL of the assay solution was aspirated from an end of each well array D and discarded. Subsequently, a sealant (mineral oil, Sigma-Aldrich) was dropped into each well array D. As a result, the well into which each of the assay solution was introduced was sealed with the sealant, and each well became each independent reaction space. After several minutes, each of the well arrays D was observed with a fluorescence microscope.

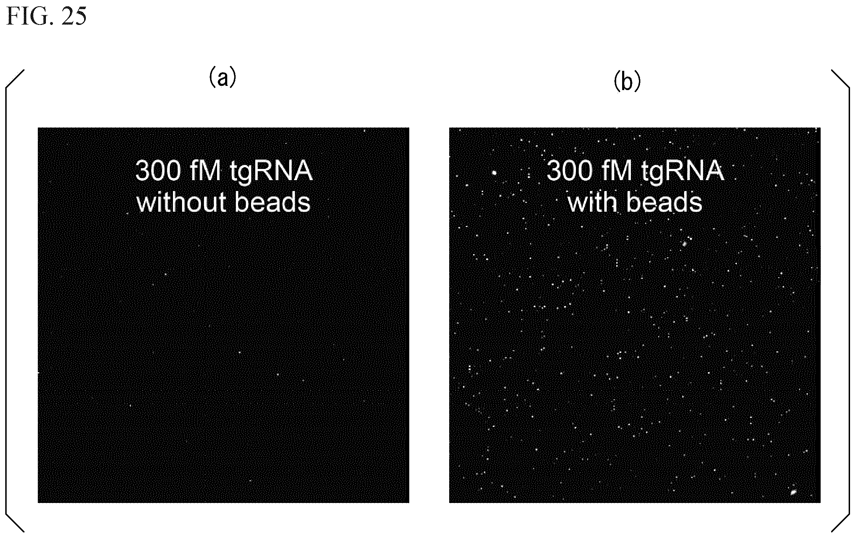

FIG. 25(a) is a representative fluorescence micrograph showing the results of an assay solution containing 300 fM of the target nucleic acid fragment and not containing the magnetic beads. FIG. 25(b) is a representative fluorescence micrograph showing the results of an assay solution containing 300 fM of the target nucleic acid fragment and the magnetic beads.

As a result, it was clarified that the detection sensitivity was significantly improved in a case where the CRISPR/Cas family protein was immobilized on the surface of the particles.

### [Industrial Applicability]

According to the present invention, it is possible to provide a technology of capable of performing detection with high sensitivity without amplifying a target nucleic acid fragment.

### [Reference Signs List]

100, 100': Three-part complex
110: CRISPR/Cas family protein
120: gRNA
130: Two-part complex
140, 140', 140": Target nucleic acid fragment
150: Substrate nucleic acid fragment
200, 1000: Fluid device
210, 510: Substrate
211: Well
212, 500, 600: Well array
220, 1010: Spacer
230, 1020: Lid member
231, 1021: Liquid inlet
310, 310': Assay solution
320: Sealant
321: Lipid
322: First lipid membrane
323: Second lipid membrane
324: Lipid bilayer membrane
330: Membrane-forming aqueous solution
520: First well
530: Second well
700, 710: Film
1030: Space
1230: Water-absorbing organic solvent
2110: Particle
2120, 2121, 2122, 2123: Single-stranded DNA fragment
F: Fluorescent substance
Q: Quencher

## Claims

1. A method for detecting a target nucleic acid fragment in a sample, comprising:
step (a) of contacting the sample with a gRNA complementary to the target nucleic acid fragment, a CRISPR/Cas family protein, and a substrate nucleic acid fragment ,
wherein the CRISPR/Cas family protein expresses nuclease activity after forming a three-part complex with the gRNA and the target nucleic acid fragment,
the CRISPR/Cas family protein is immobilized on a solid phase,
the substrate nucleic acid fragment is labeled with a fluorescent substance and a quencher,
in a case where the fluorescent substance cleaved by the nuclease activity of the three-part complex is separated from the quencher, fluorescent light is emitted by irradiation with excitation light,
the contact is performed in a reaction space having a volume of 10 aL to 100 pL, thereby forming the three-part complex in a case where the target nucleic acid fragment is present in the sample, cleaving the substrate nucleic acid fragment, and separating the fluorescent substance from the quencher; and
step (b) of irradiating the fluorescent substance with the excitation light to detect the fluorescent light,
wherein the detection of the fluorescent light indicates a presence of the target nucleic acid fragment in the sample, and
i) wherein the step (a) is performed in each well of a well array,
each well has a first well and a second well arranged at a bottom of the first well and having a smaller capacity than the first well,
the reaction space is a space inside the second well,
the CRISPR/Cas family protein is immobilized on an inner surface of the second well,
the step (a) includes
step (a1') of introducing the sample and the substrate nucleic acid fragment into each well of the well array in a case where the CRISPR/Cas family protein has formed a two-part complex with the gRNA in advance, and introducing the sample, the gRNA, and the substrate nucleic acid fragment into each well of the well array in a case where the CRISPR/Cas family protein has not formed the two-part complex with the gRNA in advance,
step (a2') of sealing each well of the well array with a sealing liquid, and
step (a3') of replacing the sealing liquid with a water-absorbing organic solvent, thereby dehydrating a content of the wells, reducing a volume, causing accumulation of the content inside the second well, forming the three-part complex in the presence of the target nucleic acid fragment in the content, cleaving the substrate nucleic acid fragment, and separating the fluorescent substance from the quencher, or
ii) wherein the CRISPR/Cas family protein is immobilized on a surface of a particle,
the method further comprising, before the step (a):
a step of mixing the sample, the gRNA, and the CRISPR/Cas family protein in a container, and forming the three-part complex including the CRISPR/Cas family protein, the gRNA, and the target nucleic acid fragment on the particle.

2. The method according to Claim 1,
wherein the sealing liquid is a fluorine-based liquid, a mineral oil, or a linear or branched, saturated or unsaturated hydrocarbon having 7 to 17 carbon atoms.

3. The method according to Claim 1 or 2,
wherein the water-absorbing organic solvent is a linear or branched, saturated or unsaturated aliphatic alcohol having 4 to 11 carbon atoms.

4. The method according to any one of Claims 1 to 3,
wherein the CRISPR/Cas family protein is a Cas12 protein or a Cas13 protein.

5. The method according to any one of Claims 1 to 4,
wherein 0 or 1 of the target nucleic acid fragment is introduced into the each reaction space.

6. The method according to any one of Claims 1 to 5,
wherein the sample is a biological sample and the step (a) is performed without purifying the target nucleic acid fragment from the biological sample.

7. A kit for detecting a target nucleic acid fragment in a sample, comprising:
a substrate having a surface on which a well having a volume of 10 aL to 100 pL is formed;
a gRNA complementary to the target nucleic acid fragment;
a CRISPR/Cas family protein immobilized on a solid phase; and
a substrate nucleic acid fragment,
wherein the CRISPR/Cas family protein expresses nuclease activity after forming a three-part complex with the gRNA and the target nucleic acid fragment,
the substrate nucleic acid fragment is labeled with a fluorescent substance and a quencher, and in a case where the fluorescent substance cleaved by the nuclease activity of the three-part complex is separated from the quencher, fluorescent light is emitted by irradiation with excitation light, and
i) wherein the well has a first well and a second well arranged at a bottom of the first well and having a smaller capacity than the first well, and the CRISPR/Cas family protein is immobilized on an inner surface of the second well, or
ii) wherein the CRISPR/Cas family protein is immobilized on a surface of a particle.

8. The kit according to Claim 7,
wherein the CRISPR/Cas family protein is a Cas12 protein or a Cas13 protein.

## Patentansprüche

1. Verfahren zum Nachweis eines Ziel-Nukleinsäurefragments in einer Probe, umfassend:
Schritt (a) des Inkontaktbringens der Probe mit einer zum Ziel-Nukleinsäurefragment komplementären gRNA, einem Protein der CRISPR/Cas-Familie und einem Substrat-Nukleinsäurefragment,
wobei das Protein der CRISPR/Cas-Familie nach Bildung eines dreiteiligen Komplexes mit der gRNA und dem Ziel-Nukleinsäurefragment Nukleaseaktivität exprimiert,
das Protein der CRISPR/Cas-Familie auf einer Festphase immobilisiert ist,
das Substrat-Nukleinsäurefragment mit einer fluoreszierenden Substanz und einem Quencher markiert ist,
falls die durch die Nukleaseaktivität des dreiteiligen Komplexes abgespaltene fluoreszierende Substanz vom Quencher getrennt wird, durch Bestrahlung mit Anregungslicht Fluoreszenzlicht emittiert wird,
der Kontakt in einem Reaktionsraum mit einem Volumen von 10 aL bis 100 pL erfolgt, wodurch der dreiteilige Komplex gebildet wird, falls das Ziel-Nukleinsäurefragment in der Probe vorhanden ist, so dass das Substrat-Nukleinsäurefragment abgespalten wird und die fluoreszierende Substanz vom Quencher getrennt wird; und
Schritt (b) der Bestrahlung der fluoreszierenden Substanz mit dem Anregungslicht zur Detektion des Fluoreszenzlichts,
wobei die Detektion des Fluoreszenzlichts das Vorhandensein des Ziel-Nukleinsäurefragments in der Probe anzeigt, und
i) wobei Schritt (a) in jedem Well einer Well-Anordnung durchgeführt wird;
jedes Well aus einem ersten Well und einem zweiten Well besteht, das am Boden des ersten Wells angeordnet ist und ein geringeres Fassungsvermögen als das erste Well hat,
der Reaktionsraum sich innerhalb des zweiten Wells befindet,
das Protein der CRISPR/Cas-Familie an einer Innenfläche des zweiten Wells immobilisiert ist,
wobei Schritt (a) umfasst
Schritt (a1') des Einbringens der Probe und des Substrat-Nukleinsäurefragments in jedes Well des Well-Arrays, sofern das Protein der CRISPR/Cas-Familie bereits einen zweiteiligen Komplex mit der gRNA gebildet hat, und des Einbringens der Probe, der gRNA und des Substrat-Nukleinsäurefragments in jedes Well des Well-Arrays, sofern das Protein der CRISPR/Cas-Familie noch keinen zweiteiligen Komplex mit der gRNA gebildet hat,
Schritt (a2') des Versiegelns jedes Wells des Well-Arrays mit einer Versiegelungsflüssigkeit, und
Schritt (a3') des Ersetzens der Versiegelungsflüssigkeit durch ein wasserabsorbierendes organisches Lösungsmittel, wodurch der Inhalt des Wells dehydriert, das Volumen reduziert, die Anreicherung des Inhalts in dem zweiten Well bewirkt, der dreiteilige Komplex in Gegenwart des Ziel-Nukleinsäurefragments im Inhalt gebildet, das Substrat-Nukleinsäurefragment gespalten und die fluoreszierende Substanz vom Quencher getrennt wird, oder
ii)wobei das Protein der CRISPR/Cas-Familie auf einer Partikeloberfläche immobilisiert ist,
wobei das Verfahren ferner vor dem Schritt (a) umfasst:
einen Schritt des Mischens der Probe, der gRNA und des Proteins der CRISPR/Cas-Familie in einem Behälter und des Bildens des dreiteiligen Komplexes, bestehend aus dem Protein der CRISPR/Cas-Familie, der gRNA und dem Zielnukleinsäurefragment, auf dem Partikel.

2. Das Verfahren nach Anspruch 1,
wobei die Versiegelungsflüssigkeit eine fluorbasierte Flüssigkeit, ein Mineralöl oder ein linearer oder verzweigter, gesättigter oder ungesättigter Kohlenwasserstoff mit 7 bis 17 Kohlenstoffatomen ist.

3. Das Verfahren nach Anspruch 1 oder 2,
wobei das wasserabsorbierende organische Lösungsmittel ein linearer oder verzweigter, gesättigter oder ungesättigter aliphatischer Alkohol mit 4 bis 11 Kohlenstoffatomen ist.

4. Das Verfahren nach einem der Ansprüche 1 bis 3,
wobei das Protein der CRISPR/Cas-Familie ein Casl2- oder ein Casl3-Protein ist.

5. Das Verfahren nach einem der Ansprüche 1 bis 4,
wobei 0 oder 1 des Ziel-Nukleinsäurefragments in jeden Reaktionsraum eingebracht wird.

6. Das Verfahren nach einem der Ansprüche 1 bis 5,
wobei die Probe eine biologische Probe ist und Schritt (a) ohne Aufreinigung des Ziel-Nukleinsäurefragments aus der biologischen Probe durchgeführt wird.

7. Kit zum Nachweis eines Ziel-Nukleinsäurefragments in einer Probe, umfassend:
einem Substrat mit einer Oberfläche, auf der ein Well mit einem Volumen von 10 aL bis 100 pL ausgebildet ist;
einer zum Ziel-Nukleinsäurefragment komplementären gRNA;
ein auf einer Festphase immobilisiertes Protein der CRISPR/Cas-Familie; und
ein Substrat-Nukleinsäurefragment
wobei das Protein der CRISPR/Cas-Familie nach Bildung eines dreiteiligen Komplexes mit der gRNA und dem Ziel-Nukleinsäurefragment Nukleaseaktivität exprimier
das Substrat-Nukleinsäurefragment mit einer fluoreszierenden Substanz und einem Quencher markiert ist, und, falls die durch die Nukleaseaktivität des dreiteiligen Komplexes abgespaltene fluoreszierende Substanz vom Quencher getrennt wird, durch Bestrahlung mit Anregungslicht Fluoreszenzlicht emittiert wird, und
i) das Well aus einem ersten Well und einem zweiten Well besteht, das am Boden des ersten Wells angeordnet ist und ein geringeres Fassungsvermögen als das erste Well hat, und das Protein der CRISPR/Cas-Familie an einer Innenfläche des zweiten Wells immobilisiert ist, oder
ii) wobei das Protein der CRISPR/Cas-Familie auf einer Oberfläche eines Partikels immobilisiert ist.

8. Das Kit nach Anspruch 7,
wobei das Protein der CRISPR/Cas-Familie ein Cas12- oder ein Cas13-Protein ist.

## Revendications

1. Méthode pour détecter un fragment d'acide nucléique cible dans un échantillon, comprenant :
une étape (a) de mise en contact de l'échantillon avec un ARNg complémentaire du fragment d'acide nucléique cible, une protéine de la famille CRISPR/Cas, et un fragment d'acide nucléique de substrat,
dans laquelle la protéine de la famille CRISPR/Cas exprime une activité de nucléase après formation d'un complexe de trois parties avec l'ARNg et le fragment d'acide nucléique cible,
la protéine de la famille CRISPR/Cas est immobilisée sur une phase solide,
le fragment d'acide nucléique de substrat est marqué avec une substance fluorescente et un désactivateur,
dans le cas où la substance fluorescente coupée par l'activité de nucléase du complexe de trois parties est séparée du désactivateur, une lumière fluorescente est émise par irradiation avec une lumière d'excitation,
le contact est effectué dans un espace réactionnel ayant un volume de 10 al à 100 pl, en formant ainsi le complexe de trois parties dans le cas où le fragment d'acide nucléique cible est présent dans l'échantillon, en coupant le fragment d'acide nucléique de substrat, et en séparant la substance fluorescente d'avec le désactivateur ; et
une étape (b) d'irradiation de la substance fluorescente avec la lumière d'excitation pour détecter la lumière fluorescente,
dans laquelle la détection de la lumière fluorescente indique une présence du fragment d'acide nucléique cible dans l'échantillon, et
i) dans laquelle l'étape (a) est effectuée dans chaque puits d'un réseau de puits,
chaque puits a un premier puits et un deuxième puits disposé au fond du premier puits et ayant une capacité inférieure à celle du premier puits,
l'espace réactionnel est un espace à l'intérieur du deuxième puits,
la protéine de la famille CRISPR/Cas est immobilisée sur une surface intérieure du deuxième puits,
l'étape (a) comprend
une étape (a1') d'introduction de l'échantillon et du fragment d'acide nucléique de substrat dans chaque puits du réseau de puits dans le cas où la protéine de la famille CRISPR/Cas a formé un complexe de deux parties avec l'ARNg à l'avance, et d'introduction de l'échantillon, de l'ARNg, et du fragment d'acide nucléique de substrat dans chaque puits du réseau de puits dans le cas où la protéine de la famille CRISPR/Cas n'a pas formé le complexe de deux parties avec l'ARNg à l'avance,
une étape (a2') de scellement de chaque puits du réseau de puits avec un liquide de scellement, et
une étape (a3') de remplacement du liquide de scellement avec un solvant organique absorbant l'eau, conduisant à la déshydratation d'un contenu des puits, la réduction d'un volume, provoquant l'accumulation du contenu à l'intérieur du deuxième puits, la formation du complexe de trois parties en présence du fragment d'acide nucléique cible dans le contenu, la coupure du fragment d'acide nucléique de substrat, et la séparation de la substance fluorescente d'avec le désactivateur, ou
ii) dans laquelle la protéine de la famille CRISPR/Cas est immobilisée sur une surface d'une particule,
la méthode comprenant en outre, avant l'étape (a) :
une étape de mélange de l'échantillon, de l'ARNg, et de la protéine de la famille CRISPR/Cas dans un récipient, et de formation du complexe de trois parties comprenant la protéine de la famille CRISPR/Cas, l'ARNg, et le fragment d'acide nucléique cible sur la particule.

2. Méthode selon la revendication 1, dans laquelle le liquide de scellement est un liquide à base de fluor, une huile minérale, ou un hydrocarbure saturé ou insaturé, linéaire ou ramifié, ayant 7 à 17 atomes de carbone.

3. Méthode selon la revendication 1 ou 2, dans laquelle le solvant organique absorbant l'eau est un alcool aliphatique saturé ou insaturé, linéaire ou ramifié, ayant 4 à 11 atomes de carbone.

4. Méthode selon l'une quelconque des revendications 1 à 3, dans laquelle la protéine de la famille CRISPR/Cas est une protéine Cas12 ou une protéine Cas13.

5. Méthode selon l'une quelconque des revendications 1 à 4, dans laquelle 0 ou 1 fragment d'acide nucléique cible est introduit dans chaque espace réactionnel.

6. Méthode selon l'une quelconque des revendications 1 à 5, dans laquelle l'échantillon est un échantillon biologique et l'étape (a) est effectuée sans purification du fragment d'acide nucléique cible à partir de l'échantillon biologique.

7. Trousse pour détecter un fragment d'acide nucléique cible dans un échantillon, comprenant :
un substrat ayant une surface sur laquelle un puits ayant un volume de 10 al à 100 pl est formé ;
un ARNg complémentaire du fragment d'acide nucléique cible ;
une protéine de la famille CRISPR/Cas immobilisée sur une phase solide ; et
un fragment d'acide nucléique de substrat,
dans laquelle la protéine de la famille CRISPR/Cas exprime une activité de nucléase après formation d'un complexe de trois parties avec l'ARNg et le fragment d'acide nucléique cible,
le fragment d'acide nucléique de substrat est marqué avec une substance fluorescente et un désactivateur, et, dans le cas où la substance fluorescente coupée par l'activité de nucléase du complexe de trois parties est séparée du désactivateur, une lumière fluorescente est émise par irradiation avec une lumière d'excitation, et
i) dans laquelle le puits a un premier puits et un deuxième puits disposé au fond du premier puits et ayant une capacité inférieure à celle du premier puits, et la protéine de la famille CRISPR/Cas est immobilisée sur une surface intérieure du deuxième puits, ou
ii) dans laquelle la protéine de la famille CRISPR/Cas est immobilisée sur une surface d'une particule.

8. Trousse selon la revendication 7, dans laquelle la protéine de la famille CRISPR/Cas est une protéine Cas12 ou une protéine Cas13.
